(19) Europäisches Patentamt
European Patent Office
Office européen des brevets

(11) Publication number: 0 370 283 B1

(12) EUROPEAN PATENT SPECIFICATION

(45) Date of publication of patent specification: 15.06.94 (51) Int. Cl.$^5$: C07K 9/00, C07K 1/06

(21) Application number: 89120267.3

(22) Date of filing: 02.11.89

# (54) Process for preparing 63-carboxyamides of teicoplanin antibiotics.

(30) Priority: 22.11.88 GB 8827202

(43) Date of publication of application:
30.05.90 Bulletin 90/22

(45) Publication of the grant of the patent:
15.06.94 Bulletin 94/24

(84) Designated Contracting States:
AT BE CH DE ES FR GB GR IT LI LU NL SE

(56) References cited:
EP-A- 0 218 099
WO-A-88/06600

(73) Proprietor: GRUPPO LEPETIT S.p.A.
Via Roberto Lepetit, 8
I-20020 Lainate (MI)(IT)

(72) Inventor: Malabarba, Adriano
5/A, Via Roma
I-20082 Binasco (MI)(IT)
Inventor: Trani, Aldo
11, Via Longhi
I-20137 Milano(IT)

(74) Representative: Macchetta, Francesco et al
GRUPPO LEPETIT S.p.A.
Patent and Trademark Department
Via Roberto Lepetit, 34
I-21040 Gerenzano (Varese) (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention is directed to a process for preparing teicoplanin amides of Formula I

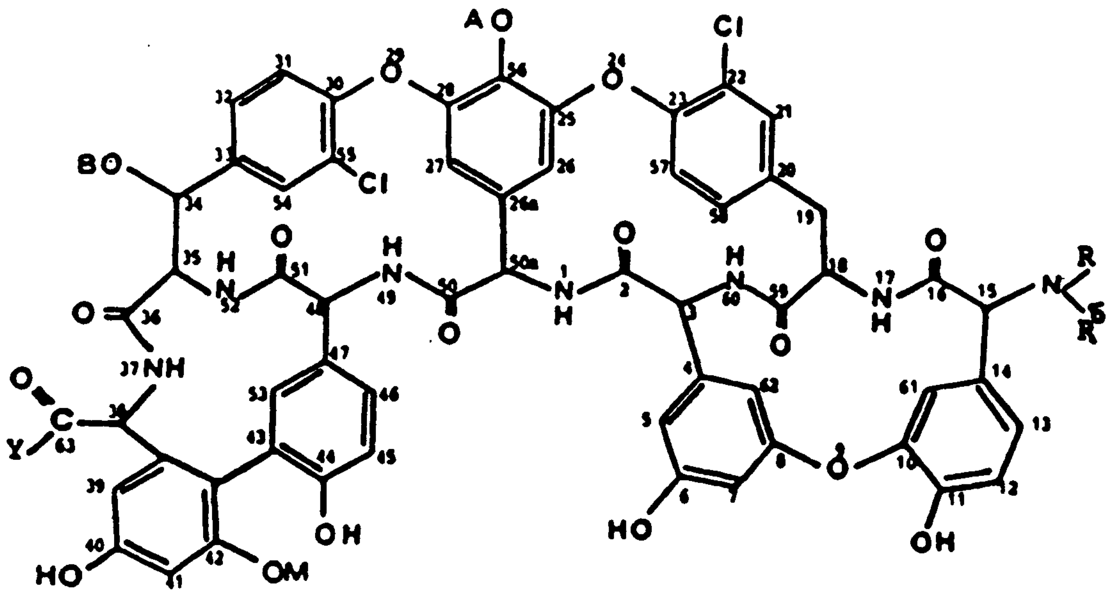

I

wherein:

R represents hydrogen; $(C_1\text{-}C_{12})$alkyl; $(C_4\text{-}C_7)$cycloalkyl, cyano $(C_1\text{-}C_3)$alkyl; $\text{-}(CH_2)_q\text{-}OOC\text{-}(C_1\text{-}C_6)$alkyl; wherein q is an integer selected from 1, 2, 3 and 4; phenyl $(C_1\text{-}C_4)$alkyl wherein the phenyl group is optionally substituted in the position ortho, meta and/or para with 1 to 3 groups selected from $(C_1\text{-}C_4)$alkyl, nitro, bromo, chloro, iodo, $(C_1\text{-}C_4)$alkoxy, and phenyl;

$R^6$ represents hydrogen, $(C_1\text{-}C_{12})$alkyl; $(C_4\text{-}C_7)$cycloalkyl; cyano $(C_1\text{-}C_3)$alkyl; $\text{-}(CH_2)_r\text{-}OOC\text{-}(C_1\text{-}C_6)$alkyl; wherein r is an integer selected from 1, 2, 3 and 4; phenyl $(C_1\text{-}C_4)$alkyl wherein the phenyl group is optionally substituted in the position ortho meta and/or para with 1 to 3 groups selected from $(C_1\text{-}C_4)$alkyl, nitro, bromo, chloro, iodo, $(C_1\text{-}C_4)$alkoxy, and phenyl;

or $R^6$ represents a group $[CHR^7\ (CR^8\ R^9)_mX]_n\text{-}R^{10}$ wherein:

$R^7$ and $R^8$ independently represent H or a $(C_1\text{-}C_6)$alkyl;

$R^9$ represents H, a $(C_1\text{-}C_6)$alkyl or OH;

$R^{10}$ represents H, a $(C_1\text{-}C_3)$alkyl, $COOR^{11}$, $OR^{11}$, $SR^{11}$, $NR^{11}R^{12}$ or halogen;

$R^{11}$ and $R^{12}$ independently represent H or a $(C_1\text{-}C_3)$alkyl; m is zero or 1, n is an integer comprised between zero and 6;

X is O, NH or a bond with the proviso that when X is O or NH, then n is different from zero, and $R^9$ is different from OH; with the proviso that when one between R and $R^6$ represents $(CH_2)_n\text{-}OOC\text{-}(C_1\text{-}C_6)$alkyl the other must represent hydrogen;

Y represents a group

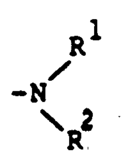

wherein

$R^1$ represents hydrogen, $(C_1\text{-}C_6)$alkyl, hydroxy-$(C_2\text{-}C_4)$alkyl, halogen $(C_2\text{-}C_4)$alkyl, $(C_1\text{-}C_4)$alkoxy-$(C_2\text{-}C_4)$alkyl, amino $(C_2\text{-}C_4)$alkyl, $(C_1\text{-}C_4)$alkylamino $(C_2\text{-}C_4)$alkyl, di$(C_1\text{-}C_4)$alkylamino $(C_2\text{-}C_4)$alkyl;

$R^2$ represents hydrogen, $(C_1\text{-}C_6)$alkyl, hydroxy-$(C_2\text{-}C_4)$alkyl, halogen $(C_2\text{-}C_4)$alkyl, $(C_1\text{-}C_4)$alkoxy-$(C_2\text{-}C_4)$alkyl, a nitrogen containing 5-6 membered heterocyclic ring which may be unsaturated, partially

saturated or wholly saturated and may contain 1 to 3 further heteroatoms selected from N, S and O wherein 1 to 3 of the ring carbons may optionally bear $(C_1-C_4)$alkyl substituents and one of the ring nitrogens may optionally bear a substituent $R^5$ selected from $(C_1-C_4)$alkyl, $(C_4-C_7)$cycloalkyl, phenyl optionally substituted with halogen or $(C_1-C_4)$alkyl, phenyl $(C_1-C_4)$alkyl, pyridyl, $(C_1-C_4)$alkylpyridinio, and when the ring is wholly saturated two of the ring members may optionally be bridged by an alkylene chain of 1 to 3 carbon atoms wherein one of the methylene groups may optionally be replaced by -NH- or -N[$(C_1-C_4)$alkyl]; a group -alk-W wherein "alk" represents a linear alkylene chain of 1 to 8 carbon atoms which is optionally substituted with a substituent selected from $(C_1-C_4)$alkyl, hydroxy-$(C_1-C_4)$alkyl, hydroxy, carboxyaminocarbonyl, $(C_1-C_4)$alkylaminocarbonyl, di$(C_1-C_4)$alkylaminocarbonyl, $(C_1-C_4)$alkylcarbonyl, phenyl$(C_1-C_4)$alkoxycarbonyl, and W represents a carboxy, $(C_1-C_4)$alkoxycarbonyl, phenyl-$(C_1-C_4)$alkoxycarbonyl, aminocarbonyl, $(C_1-C_4)$-alkylaminocarbonyl, di$(C_1-C_4)$alkylaminocarbonyl, pentosaminocarbonyl, hexosaminocarbonyl, ureido, guanidino, a nitrogen containing 5-6 membered heterocyclic ring defined as above, a group of the formula

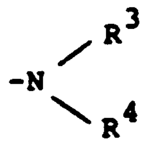

wherein $R^3$ and $R^4$ each independently represents hydrogen, $(C_1-C_6)$alkyl, hydroxy $(C_2-C_4)$alkyl and halogen $(C_2-C_4)$alkyl or $R^4$ represents phenylmethyloxycarbonyl and $R^3$ represents hydrogen; or $R^1$ and $R^2$ taken together with the adjacent nitrogen atom represent a saturated 5-7 membered heterocyclic ring which may optionally bear one to two $(C_1-C_4)$alkyl substituents on the ring carbons and may contain a further heterogroup selected from -O-,-S-, and -$NR^5$-wherein $R^5$ is defined as above;

A represents hydrogen or -N[$(C_9-C_{12})$aliphatic acyl]-beta-D-2-deoxy-2-amino-glucopyranosyl;

B represents hydrogen or N-acetyl-beta-D-2-deoxy-2-aminoglucopyranosyl;

M represents hydrogen or alpha-D-mannopyranosyl; with the proviso that B represents hydrogen only when A and M are simultaneously hydrogen, and with the further proviso that when W represents a group

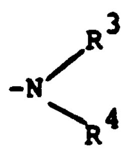 ,

ureido, guanidino or a nitrogen containing 5-6 membered heterocyclic ring as defined above directly connected with the "alk" moiety through a bond with a ring nitrogen atom, the linear alkylene "alk" moiety must be of at least two carbon atoms; and the pharmaceutically acceptable addition salts thereof; which comprises reacting a compound of Formula II

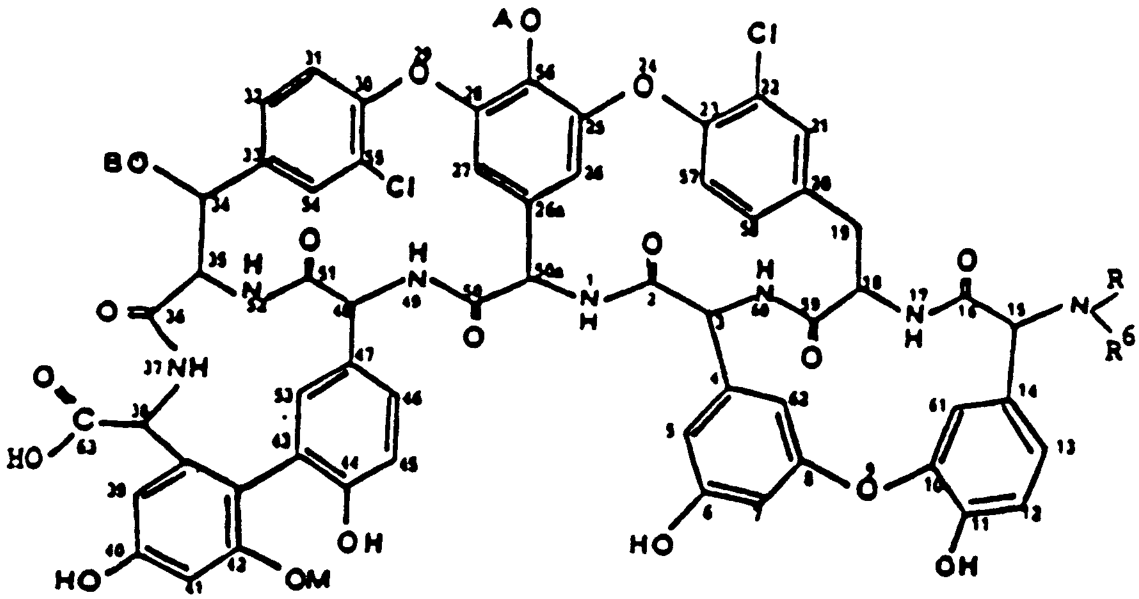

II

wherein:

A, B, M, R, and $R^6$ are as defined, protected, if necessary, at the 15 amino function by a protecting group, with an activated ester forming reagent to give an activated ester function which is substituted by an amine of formula $NHR^1R^2$ wherein $R^1$ and $R^2$ are as defined, by contacting with an excess of said amino in an organic polar solvent at a temperature comprised between 5 °C and 60 °C.

Teicoplanin is the international non-proprietary name (INN) of the antibiotic substance formerly named Teichomycin which is obtained by cultivating the strain Actinoplanes teichomyceticus nov. sp. ATCC 31121 in a culture medium containing assimilable sources of carbon, nitrogen and inorganic salts (see U.S. Patent No. 4,239,751).

According to the procedure described in the above cited patent an antibiotic complex containing Teichomycin $A_1$, $A_2$ and $A_3$ is recovered from the separated fermentation broth by extraction with a suitable water insoluble organic solvent and precipitation from the extracting solvent according to common procedures. Teichomycin $A_2$, which is the major factor of the isolated antibiotic complex, is then separated from the other factors by means of column chromatography on Sephadex®. British Patent No. 2121401 discloses that antibiotic Teichomycin $A_2$ actually is a mixture of five closely related co-produced main components.

According to recent structural studies it is possible to represent teicoplanin $A_2$ (formerly Teichomycin $A_2$) main components 1, 2, 3, 4 and 5 by the above formula I wherein R and $R^6$ are hydrogen, Y is hydroxy, A represents -N[($C_{10}$-$C_{11}$)aliphatic acyl]-beta-D-2-deoxy-2-aminoglucopyranosyl, B represents N-acetyl-beta-D-2-deoxy-2-amino-glucopyranosyl, M represents alpha-D-manno-pyranosyl.

More particularly, in teicoplanin $A_2$ component 1, the [$C_{10}$-$C_{11}$)-aliphatic acyl] substituent represents Z-decenoyl, in teicoplanin $A_2$ component 2 represents 8-methylnonanoyl, in teicoplanin $A_2$ component 3 represents decanoyl, in teicoplanin $A_2$ component 4 represents 8-methyldecanoyl, in teicoplanin $A_2$ component 5 represents 9-methyldecanoyl.

European Patent Application Publication No. 306645 describes production of teicoplanin compounds where the aliphatic acid group of the beta-D-2-deoxy-2-amino glucopyranosyl moiety is a n-nonanoyl (compound B) or a 6-methyloctanoyl group (compound A).

In the paper entitled: "isolation by HPLC and structural determination of minor components of teicoplanin" given by Zanol et al., at the 17th International Symposium on chromatography, wien, September 25-30, 1988, other two teicoplanin compounds (RS1 and RS2) are described.

Said compounds are characterized in that the aliphatic acyl moieties of the beta-D-2-deoxy-2-amino glucopyranosyl moiety are respectively methylundecanoyl and dodecanoyl.

All the sugar moieties, when present, are linked to the teicoplanin nucleus through O-glycosidic bonds.

In addition, it has been found that it is possible to transform teicoplanin, a pure factor thereof or a mixture of any of said factors in any proportion, into unitary antibiotic products by means of selective hydrolysis of one or two sugar moieties. They are named antibiotic L 17054 and antibiotic L 17046 and are described in European Patent Application Publication No. 119575 and European Patent Application Publication No. 119574, respectively.
Preferred hydrolysis conditions for the production of antibiotic L 17054 are: 0.5 N hydrochloric acid at a temperature between 70 °C and 90 °C and for a time which is generally between 15 and 90 min.
Antibiotic L 17054 is represented by the above formula I wherein Y is hydroxy, R, $R^6$ and A represent hydrogen, B represents N-acetyl-beta-D-2-deoxy-2-amino-glucopyranosyl, M represents alpha-D-mannopyranosyl wherein the sugar moieties are linked to the peptidic nucleus through an O-glycosidic bond.
Preferred hydrolysis conditions for the preparation of antibiotic L 17046 are: 1-3 N hydrochloric acid, at a temperature between 50° and 90 °C and for a time which is generally between 30 and 60 min.
Antibiotic L 17046 is represented by the above formula I wherein Y is hydroxy, R, $R^6$, A and M represent hydrogen atoms, and B is N-acetyl-beta-D-2-deoxy-2-amino-glucopyranosyl wherein the sugar moiety is linked to the peptidic nucleus through an O-glycosidic bond.

EP-A- publication No. 301247 describes de-mannosyl teicoplanin derivatives, i.e. compounds of the formula I above wherein A and B are different from hydrogen, M is hydrogen and Y is hydroxy.

The complete selective cleavage of all the sugar moieties of the teicoplanin compounds gives an aglycone molecule which is called antibiotic L 17392, or deglucoteicoplanin, and is represented by the above formula I wherein Y is hydroxy, and R, $R^6$, A, B, and M each individually represents a hydrogen atom. This selective hydrolysis process is described in European patent application publication No. 146053.

A substance having the same structural formula is disclosed in European Patent Application Publication No. 0090578 and is named antibiotic A 41030 factor B. This substance is obtained by means of a microbiological process which involves the fermentation of the strain Streptomyces virginiae NRRL 12525 or Streptomyces virginiae NRRL 15156 in a suitable medium, the isolation, purification and separation into its components of antibiotic A 41030, an antibiotic complex of at least seven factors, antibiotic A 41030 factor B included.

All the above named compounds, i.e. teicoplanin, teicoplanin $A_2$ complex, teicoplanin $A_2$ component 1, teicoplanin $A_2$ component 2, teicoplanin $A_2$ component 3, teicoplanin $A_2$ component 4, teicoplanin $A_2$ component 5, antibiotic L 17054, antibiotic L 17046, antibiotic L 17392, "compound A or RS3", "compound B or RS4", RS1, RS2 and any mixture thereof in any proportion, are suitable starting materials for the preparation of the amide derivatives of the invention. In the present specification "teicoplanin compound" or "teicoplanin starting material" is used to indicate any one of the above starting materials, i.e. teicoplanin as obtained according to U.S. patent 4,239,751, any further purification thereof, teicoplanin $A_2$ complex, a compound of the above formula II wherein R and $R^6$ are both hydrogen, Y is hydroxy, A represents hydrogen or -N[($C_9$-$C_{12}$)aliphatic acyl]-beta-D-2-deoxy-2-amino-glucopyranosyl, B represents hydrogen or N-acetyl-beta-D-2-deoxy-2-amino-glucopyranosyl, M represents hydrogen or alpha-D-mannopyranosyl, with the proviso that B may represent hydrogen only when A and M are simultaneously hydrogen, a salt thereof, or a mixture thereof in any proportion.

The compounds of formula I wherein $R^6$ and R are hydrogen atoms and a process for preparing them are disclosed in the European Patent Application Publication No. 218099. The process of the EP-A-218099 comprises reacting a teicoplanin starting material as defined or a compound of formula II wherein R and $R^6$ are hydrogen atoms with a molar excess of an amine of formula $HNR^1R^2$, wherein $R^1$ and $R^2$ are defined as above and wherein the reactive functions, other than the amino function to be reacted with the carboxyl moiety of the teicoplanin starting material, are protected by means of known per se protecting groups, in an inert organic solvent, and in the presence of a slight molar excess of a condensing agent selected from ($C_1$-$C_4$)alkyl, phenyl or heterocyclic phosphorazidates such as diphenyl phosphorazidate (DPPA), diethyl phosphorazidate, di(4-nitrophenyl)phosphorazide, dimorpholylphosphorazide and diphenylphosphorochloridate, at a temperature between 0° and 20 °C.

Furthermore an amide compound thus obtained can be optionally transformed in other teicoplanin compounds by known reactions such as for example by selective hydrolysis of the sugar moieties with strong acids in an organic polar aprotic solvent to obtain a compound of formula I wherein A, B and M are hydrogen atoms. Surprisingly, with the process of the present invention we obtain yields of the final products remarkably higher than the yields of the corresponding products obtained by the previous process described in EPA Publication No. 218099.

As used herein the term "alkyl", either alone or in combination with other substituents, includes both straight and branched hydrocarbon groups; more particularly, "($C_1$-$C_6$)alkyl" represents a straight or branched aliphatic hydrocarbon chain of 1 to 6 carbon atoms such as methyl, ethyl, propyl, 1-methylethyl, butyl, 1-methylpropyl, 1,1-dimethylethyl, pentyl, 1-methylbutyl, 2-methylbutyl, 1-hexyl, 2-hexyl, 3-hexyl, 3,3-dimethyl-1-butyl, 4-methyl-1-pentyl and 3-methyl-1-pentyl; likewise, "($C_1$-$C_4$)alkyl" represents a straight or branched hydrocarbon chain of 1 to 4 carbon atoms such as those alkyl of 1 to 4 carbons exemplified above.

The term "halogen" represents an halogen atom selected from fluorine, chlorine, bromine and iodine. The pentosamino moieties of the pentosaminocarbonyl substituent are 2- or 3-amino (2- or 3-deoxy) either D or L or D, L pentose groups in either anomeric form or in an anomeric mixture, such as 2- or 3-amino(2- or 3-deoxy)-ribose, 2- or 3-amino(2- or 3-deoxy)arabinose, 2- or 3-amino(2- or 3-deoxy)xylose and 2- or 3-amino (2 or 3-deoxy)lyxose.

The hexosamino moieties of the hexosaminocarbonyl substituent are either D or L, or D, L 2- or 3-amino (2- or 3-deoxy)hexose groups in either anomeric form or in an anomeric mixture such as 2- or 3-amino(2- or 3-deoxy)allose, 2- or 3-amino(2- or 3-deoxy)altrose, 2- or 3-amino(2- or 3-deoxy)glucose, 2- or 3-amino(2-or 3-deoxy)mannose, 2- or 3-amino(2- or 3-deoxy)galactose, and 3-or 4-amino(3- or 4-deoxy)-fructofuranose.

"Linear alkylene chains of 1 to 8 carbon atoms" as defined in the present application are straight alkylene chains of 1, 2, 3, 4, 5, 6, 7 or 8 carbon atoms. Representative examples of linear alkylene chains of 1 to 8 carbon atoms are:

$-CH_2-$

$-CH_2-CH_2-$

$-CH_2-CH_2-CH_2-$

$-CH_2-CH_2-CH_2-CH_2-$

$-CH_2-CH_2-CH_2-CH_2-CH_2-$

$-CH_2-CH_2-CH_2-CH_2-CH_2-CH_2-$

$-CH_2-CH_2-CH_2-CH_2-CH_2-CH_2-CH_2-$

$-CH_2-CH_2-CH_2-CH_2-CH_2-CH_2-CH_2-CH_2-$

These linear alkylene chains optionally may bear substituents as described above.

The expression "a nitrogen containing 5-6 membered heterocyclic ring which may contain 1 to 3 further heteroatoms selected from N, S and O" according to the present invention includes unsaturated, partially saturated and wholly saturated ring systems such as pyridinyl, pyrimidinyl, pyrazinyl, pyrrolidinyl, piperidinyl, piperazinyl, oxazolyl, oxazolinyl, oxazolidinyl, pyrazolinyl, pyrazolidinyl, thiazolidinyl, morpholinyl, thiomorpholinyl, pyrrolyl, pyrrolinyl, imidazoyl, imidazolidinyl, thiadiazolyl, oxadiazolyl, and tetrazolyl.

In said "nitrogen containing 5-6 membered heterocyclic ring" 1 to 3 ring carbons may optionally bear $(C_1-C_4)$alkyl substituents defined as above. When a ring carbon is saturated, it may be simultaneously substituted with two $(C_1-C_4)$alkyl groups.

When the above defined "nitrogen containing 5-6 membered heterocyclic ring" is a wholly saturated ring, this definition includes also those heterocyclic rings which have two ring members bridged by an alkylene chain of 1 to 3 carbon atoms wherein a methylene group may optionally be replaced by a group -NH- or $-N[(C_1-C_4)alkyl]$. Examples of said bridged rings are the following:

1-azabicyclo[2.2.2]octane, 1,4-diazabicyclo[3.2.2] nonane, 1-azabicyclo[2.2.1]heptane, 1-azabicyclo[3.2.1]-octane, 8-azabicyclo[3.2.1]octane, 3-azabicyclo[3.2.1]octane, 1-azabicyclo[3.3.1]nonane, 9-azabicyclo[3.3.1]-nonane, 3,8-diazabicyclo[3.2.1] octane, 2-azabicyclo[2.2.1]heptane, 2-azabicyclo-[2.2.2]octane, 3-azabicyclo-[3.2.2]nonane.

Accordingly, representative compounds of this invention include those of the general formula above where the symbol

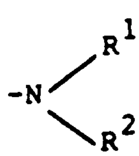

represents a substituent derived from one of the following moieties:

1-azabicyclo[2.2.2]octan-3-amine,

1-azabicyclo[2.2.2]octan-2-amine,

1-azabicyclo[2.2.2]octan-3-amine, 6-methyl

1-azabicyclo[2.2.2]octan-3-amine, N-methyl

1-azabicyclo[2.2.2]octan-3-ethanamine,

1-azabicyclo[2.2.2]octan-4-amine,

1-azabicyclo[2.2.2]octan-4-amine, N-methyl

1-azabicyclo[2.2.2]octan-2-methanamine,

1-azabicyclo[2.2.1]heptan-3-amine
1-azabicyclo[3.2.1]octan-3-methanamine,
8-azabicyclo[3.2.1]octan-3-amine, 8-methyl
8-azabicyclo[3.2.1]octan-3-amine, 8-ethyl
8-azabicyclo[3.2.1]octan-2-methanamine,
3-azabicyclo[3.2.1]octan-3-ethanamine,
1-azabicyclo[3.3.1]nonan-4-amine
1-azabicyclo[3.3.1]nonan-3-methanamine
9-azabicyclo[3.3.1]nonan-3-amine, 9-methyl
2-azabicyclo[2.2.1]heptan-5-amine, 2-methyl
2-azabicyclo[2.2.2]octan-5-amine, 2-methyl

The expression "a saturated 5-7 membered heterocyclic ring which may optionally bear one to two ($C_1$-$C_4$)alkyl substituents on the ring carbons and may optionally contain a further heterogroup selected from -O-, -S- and -$NR^5$-" includes, for instance, the following heterocyclic groups: morpholinyl, piperidinyl, piperazinyl, thiomorpholinyl, pyrazolidinyl, 1,3-oxazolidinyl, 1,3-thiazolidinyl and hexahydroazepinyl, which may optionally be substituted by one or two ($C_1$-$C_4$)alkyl group on the carbon skeleton.

European Patent Application Publication No. 276740 discloses teicoplanin $N^{15}$-alkyl and $N^{15}$, $N^{15}$-dialkyl derivatives wherein R is as defined and $R^6$ has the same meaning of R, and their method of manufacture.

These alkyl derivatives fall within formula II above and are conveniently used as starting materials in the process of the present invention. A preferred group of compounds which can be prepared with the process of the invention is represented by those compounds of formula I wherein R represents a hydrogen atom and the other substituents are as defined above.

A further preferred group of compounds which can be prepared with the process of the present invention are those compounds of formula I wherein R and $R^6$ represent hydrogen and the other substituents are as above defined.

A further preferred group of compounds which can be prepared with the process according to the present invention is represented by those compounds of formula I wherein

R represents hydrogen, $R^6$ is as defined above

Y represents a group

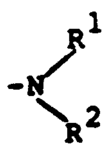

wherein

$R^1$ represents hydrogen or ($C_1$-$C_6$)alkyl,

$R^2$ represents ($C_1$-$C_6$)alkyl, a nitrogen containing 5-6 membered heterocyclic ring which may be unsaturated, partially saturated or wholly saturated and may contain from 1 to 3 further heteroatoms selected from N, S and O wherein 1 to 3 of the ring carbons may optionally bear ($C_1$-$C_4$)alkyl substituents and one of the ring nitrogens may optionally bear a substituent $R^5$ selected from ($C_1$-$C_4$)alkyl, ($C_4$-$C_7$)cycloalkyl, phenyl, and pyridyl;

a wholly saturated nitrogen containing 5-6 membered heterocyclic ring which may contain a further N atom wherein 1 to 3 of the ring carbons may optionally bear ($C_1$-$C_4$)alkyl substituents, one of the ring nitrogens may optionally bear a substituent $R^5$ representing ($C_1$-$C_4$)alkyl and two of the ring members are bridged by an alkylene chain of 1 to 3 carbon atoms wherein one of the methylene groups may optionally be replaced by -NH- or -N[($C_1$-$C_4$)alkyl];a group -alk-W wherein "alk" represents a linear alkylene chain of 1 to 8 carbon atoms which is optionally substituted with a substituent selected from ($C_1$-$C_4$)alkyl, carboxy, aminocarbonyl, ($C_1$-$C_4$)alkylaminocarbonyl, di($C_1$-$C_4$)alkylaminocarbonyl, ($C_1$-$C_4$)alkoxycarbonyl, phenyl($C_1$-$C_4$)alkoxycarbonyl, and W represents a carboxy, ($C_1$-$C_4$)alkoxycarbonyl, phenyl($C_1$-$C_4$)alkoxycarbonyl, aminocarbonyl, ($C_1$-$C_4$)aminocarbonyl, di($C_1$-$C_4$)aminocarbonyl, glucosaminocarbonyl, ureido, guanidino, a nitrogen containing 5-6 membered heterocyclic ring which may be unsaturated, partially saturated or wholly saturated and may contain 1 to 3 further heteroatoms selected from N, S and O wherein 1 to 3 of the ring carbons may optionally bear ($C_1$-$C_4$)alkyl substituents and one of the ring nitrogens may optionally bear a substituent $R^5$ selected from ($C_1$-$C_4$)alkyl, ($C_4$-$C_7$)cycloalkyl, phenyl, and pyridyl; a wholly saturated nitrogen containing 5-6 membered heterocyclic ring which may contain a further N atom wherein 1 to 3 of the ring carbons may optionally bear ($C_1$-$C_4$)alkyl substituents, one of the ring nitrogens may optionally

bear a substituent $R^5$ representing $(C_1-C_4)$alkyl and two of the ring members are bridged by an alkylene chain of 1 to 3 carbon atoms wherein one of the methylene groups may optionally be replaced by -NH- or $-N[(C_1-C_4)$alkyl]; a group of the formula

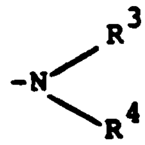

wherein:

$R^3$ and $R^4$ each independently represent hydrogen, $(C_1-C_6)$alkyl, hydroxy$(C_2-C_4)$alkyl and halogeno$(C_2-C_4)$alkyl, or $R^4$ represents phenylmethyloxycarbonyl and $R^3$ represents hydrogen; or $R^1$ and $R^2$ taken together with the adjacent nitrogen atom represent a saturated 5-7 membered heterocyclic ring which may optionally bear one to two $(C_1-C_4)$alkyl substituents on the ring carbons and may contain a further heterogroup selected from -O-, -S-, and $-NR^5-$ wherein $R^5$ is defined as above;

A represents hydrogen or $-N[(C_{10}-C_{12})$aliphatic acyl] beta-D-2-deoxy-2-amino-glucopyranosyl,

B represents hydrogen or N-acetyl-beta-D-2-deoxy-2-amino-glucopyranosyl,

M represents hydrogen or alpha-D-mannopyranosyl;

with the proviso that B represents hydrogen only when A and M are simultaneously hydrogen and with the further proviso that when W represents

a group

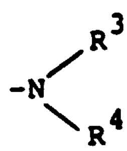

,

ureido, guanidino or a nitrogen containing 5-6 membered heterocyclic ring as defined above directly connected with the "alk" moiety through a bond with a ring nitrogen atom, the linear alkylene "alk" moiety must be of at least two carbon atoms; and the addition salts thereof.

Other preferred compounds which are prepared with the process of the invention are represented by those compounds of formula I wherein R is hydrogen or methyl, $R^1$, $R^6$, A, B and M are as defined and $R^2$ represents a group

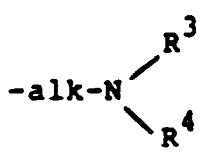

wherein "alk" is a linear alkylene chain of 2 to 8 carbon atoms and $R^3$ and $R^4$ independently represent H or $(C_1-C_6)$alkyl groups and the pharmaceutically acceptable addition salts thereof.

A further group of preferred compounds which can be prepared according to the process of the invention are those compounds of formula I wherein R represents hydrogen; $R^1$ represents hydrogen or $(C_1-C_4)$alkyl, $R^2$ represents a wholly saturated nitrogen containing 5-6 membered heterocyclic ring which may contain a further N atom wherein 1 to 3 of the ring carbons may optionally bear $(C_1-C_4)$alkyl substituents, one of the ring nitrogens may optionally bear a substituent $R^5$representing $(C_1-C_4)$alkyl and two of the ring members are bridged by an alkylene chain of 1 to 3 carbon atoms wherein one of the methylene groups may optionally be replaced by -NH- or $-N[(C_1-C_4)$alkyl];or a group -alk-W wherein alk represents a linear alkylene chain of 1 to 3 carbon atoms and W is a wholly saturated nitrogen containing 5-6 membered heterocyclic ring defined as in the paragraph immediately above.

Another group of preferred compounds of the invention is represented by those compounds wherein A, B, and M either represent the sugar moieties as above defined or each simultaneously represents a hydrogen atom.

Other most preferred compounds which are prepared with this process are those of formula I wherein A, B and M either simultaneously represent the sugar moieties defined above or each simultaneously represents a hydrogen atom, R represents hydrogen, and $NR^1R^2$ represents a group -HN(alk)W wherein "alk" represents a linear alkylene chain of 2, 3, 4, 5, 6, 7 or 8 units and W represents a group selected from: $-NH_2$, $-NHCH_3$, $-NHC_2H_5$, $-N(CH_3)_2$, $-N(C_2H_5)_2$, and $-N(CH_3)(C_2H_5)$, or a group $-HNCH(COOCH_3)-(CH_2)_4NH_2$, or

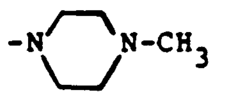 .

Another group of compounds which can be conveniently prepared according to the process of the present invention is represented by those compounds of formula I wherein R, A, B and M are as defined, $R^2$ represents a group

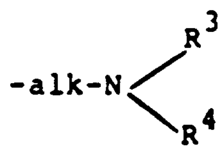

wherein:

"alk" is a linear alkylene chain of 2 to 8 carbon atoms, $R^3$ and $R^4$ independently represent H or a $(C_1-C_6)$ alkyl group, R is H or methyl and $R^6$ represents a group in which n ranges from 1 to 3, m is zero or 1, $R^7$ and $R^8$ represent H or $(C_1-C_6)$alkyl, $R^9$ represents H, $CH_3$, or OH; $R^{10}$ represents H, $OR^{11}$ or $NR^{11}R^{12}$; $R^{11}$ and $R^{12}$ independently represents H or $CH_3$; X is O or a bond with the proviso that when X is O, n is 1 and $R^9$ is H or $CH_3$.

A particularly preferred group of compounds which are conveniently prepared according to the process of the present invention is represented by those novel compounds not specifically disclosed in the already cited European Patent Application Publication No. 218099 i.e. those teicoplanin amide compounds of formula I wherein:

a) A is N[$(C_9-C_{11})$aliphatic acyl]-beta-D-2-deoxy-2-aminoglucopyranosyl;
B is N-acetyl-beta-D-2-deoxy-2-aminoglucopyranosyl;
M is alpha-D-mannopyranosyl
R and $R^6$ are hydrogen atoms and
Y is $NH_2$, $NHCH_3$, $NH(CH_2)_2NH_2$, $NH(CH_2)_3NH_2$, $NH(CH_2)_2NHCH_3$, $NH(CH_2)_3NHCH_3$, $NH(CH_2)_2NH-(C_2H_5)$, $NH(CH_2)_3NH(C_2H_5)$, $N(CH_3)(CH_2)_2NHCH_3$, $N(CH_3)(CH_2)_3NHCH_3$, $NHCH(COOCH_3)(CH_2)_4NH_2$, $NH(CH_2)_3NH(CH_2)_2OH$ or $NH(CH_2)_3N(CH_3)_2$;
b) A is N[$(C_9-C_{11})$aliphatic acyl]-beta-D-2-deoxy-2-aminoglucopyranosyl
B is N-acetyl-beta-D-2-deoxy-2-aminoglucopyranosyl
M is alpha-D-mannopyranosyl
R is hydrogen, $R^6$ is $CH_2CH_2-N(CH_3)_2$ and
Y is $NH(CH_2)_3N(CH_3)_2$
c) A is N-(8-methylnonanoyl)-beta-D-2-deoxy-2-aminoglucopyranosyl
B is N-acetyl-beta-D-2-deoxy-2-aminoglucopyranosyl
M is alpha-D-mannopyranosyl
R and $R^6$ are hydrogen atoms and
Y is $NH(CH_2)_3N(CH_3)_2$
d) A, B, M, R and $R^6$ are hydrogen atoms and
Y is $NH_2$, $NHCH_3$, $NH(CH_2)_2NH_2$, $NH(CH_2)_3NH_2$, $NH(CH_2)_4NH_2$, $NH(CH_2)_6NH_2$, $NH(CH_2)_2NHCH_3$, $NH-(CH_2)_3NHCH_3$, $NH(CH_2)_2NHC_2H_5$, $NH(CH_2)_5N(CH_3)_2$, $NH(CH_2)_7N(CH_3)_2$, $N(CH_3)(CH_2)_2NHCH_3$, $N(CH_3)-(CH_2)_3NHCH_3$ and $NH(CH_2)_3NH(CH_2)_2OH$;
e) A, B, M and R are hydrogen atoms, $R^6$ is a $CH(CH_3)CH(CH_3)OH$ and Y is $NH(CH_2)_3-N(CH_3)_2$;

and the pharmaceutically acceptable salts thereof. Among the compounds above described the preferred one is the $C^{63}$-3-(dimethylamino)-1-propylamide teicoplanin $A_2$ component 2, which may be both in the free base form and in salt form (for instance as monoacetate, hydrochloride or di-hydrochloride).

The starting compounds of the formula II can be single compounds corresponding to said formula or a mixture thereof, as resulting from the teicoplanin starting compound (as defined above) utilized for the manufacture of said compounds of formula II.

Accordingly, the resulting final compound of formula I could be a mixture of products corresponding to the mixture used as the starting material. Said mixture of products obtained according to the process of the invention can be used as such or separated into its single components according to the procedures described in British Patent 2121401, and in European Patent Publication Nos. 276740 and 218099. The compounds prepared with the process of the present invention are useful as semi-synthetic antibacterial agents mainly active against gram positive bacteria but also active against gram negative bacteria. In some instances the compounds obtained according to this process can be used as intermediates or starting materials for the manufacture of other semi synthetic teicoplanin derivatives, for instance those described in our European Pat. Appln. Serial No.89112608.

In the case it is desired to prepare the compounds of formula I wherein R and $R^6$ are hydrogen atoms and the other substituents are as above defined the general procedure comprises reacting a teicoplanin starting material or a compound of formula II wherein R and $R^6$ are both hydrogen, or a teicoplanin protected on the 15-amino function with an activated ester forming reagent to form an "activated" ester function that can be easily substituted by an amine of formula $NHR^1R^2$ to give the corresponding amide of formula I.

The $N^{15}$-amino function can be protected by methods known per se in the art such as those described in reference books like T.W. Greene, "Protective Groups in Organic Synthesis", John Wiley and Sons, New York, 1981, and M. Mc Omie "Protecting Groups in Organic Chemistry" Plenum Press, New York, 1973. These protecting groups must be stable at the conditions of the reaction process, must not unfavorably interfere with the main amidation reaction, and must be easily cleavable and removable from the reaction medium at the end of the reaction without altering the newly formed amide bond.

Representative examples of N-protecting groups which may be advantageously used in the process of the invention for protecting an amino function both in the teicoplanin starting material and, when appropriate, in the $R^1$ and $R^2$ moiety of the amine $HNR^1R^2$ are carbamate forming reagents characterized by the following oxycarbonyl groups: 1,1-dimethylpropynyloxycarbonyl, t-butyloxycarbonyl, vinyloxycarbonyl, aryloxycarbonyl, cinnamyloxycarbonyl, benzyloxycarbonyl, p-nitrobenzyloxycarbonyl, 3,4-dimethoxy-6-nitrobenzyloxycarbonyl, 2,4-dichlorobenzyloxycarbonyl, 5-benzisoxazolylmethyloxycarbonyl, 9-anthranylmethyloxycarbonyl, diphenylmethyloxycarbonyl, isonicotinyloxycarbonyl, S-benzyloxycarbonyl, and the like. Other suitable N-protecting agents are aldehydes or ketones, or derivatives thereof which are capable of forming Schiff bases with the amino group of the teicoplanin nucleus to be protected.

Preferred examples of such Schiff base forming agents are benzaldehydes and particularly preferred is 2-hydroxybenzaldehyde (salicylaldehyde).

A convenient means of protection in the case the amine reactant $HNR^1R^2$ contains a primary amino function as substituent for $R^1$ and/or $R^2$, is, in some instances, the formation of a benzyliden derivative which may be prepared by reacting the amine $HNR^1R^2$ with benzaldehyde in a lower alkanol, such as ethanol, preferably at room temperature. After the reaction with the selected teicoplanin starting material has been completed, the benzylidene protecting group may be removed as known in the art, e.g. by treatment with diluted mineral acid, preferably hydrochloric acid, at room temperature.

Obviously, when the final compound of formula I contains groups which are labile under acidic conditions, e.g. when A, B or M represent sugar moieties as above defined which may be hydrolized in an acidic medium, other removal conditions must be used, such as catalytic hydrogenation using for instance palladium on carbon as the catalyst to remove the proper protecting group.

In this case, however, attention should be paid to the presence of groups which may be modified by catalytic hydrogenation. A typical consequence of the catalytic hydrogenation of a derivative of formula I wherein A represents a group as above defined whose acyl portion is Z-decenoyl (i.e. a teicoplanin $A_2$ component 1 derivative or a mixture containing it) is that it is at least partially transformed into the corresponding decanoyl derivative (i.e. a derivative of teicoplanin $A_2$ component 3).

As it is appreciated by the skilled technician, the ultimate choice of the specific protecting group depends on the characteristics of the particular amide derivative which is desired. In fact, this amide function of the final compound should be stable at the conditions of removal of the protecting group(s).

Since the conditions of removal of the different protecting groups are known, the skilled technician is capable of selecting the proper protecting group. For instance, where the final compound possesses also a benzyl ester function or N-benzyl function, the protecting groups which are usually removable by catalytic hydrogenation, such as the benzyloxycarbonyl group, should be avoided, while those protecting groups which are removable under acidic conditions, such as t.butoxycarbonyl, can be conveniently used. On the

contrary, catalytic hydrogenation may be conveniently used in a case like the above when it is desired to convert a compound of formula I containing said N-benzyl or benzyl ester function in the $-NR^1R^2$ moiety into the corresponding compound wherein said N-benzyl or benzyl ester function is replaced by a hydrogen atom.

The formation of "activated" esters is described in general terms in Fieser and Fieser, Reagent for organic synthesis, pages 129-130.

Examples of said activated ester forming reagents that can be conveniently used in the process of the invention are those described by R. Schwyzer et al. in Helv. Chim. Acta, 1955, 38, 69-70 and encompass: $ClCH_2CN$, $BrCH_2COOC_2H_5$, $BrCH(COOC_2H_5)_2$, $ClCH_2COCH_3$,

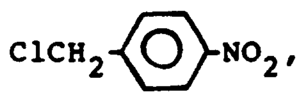, $ClCH_2CH_2N(C_2H_5)_2$

A preferred reagent of this type is chloroacetonitrile. In this case chloroacetonitrile itself or dimethyl formamide can be used as preferred solvents.

Inert organic solvents useful for the formation of "activated" esters are those organic aprotic solvents which do not unfavorably interfere with the reaction course and are capable of at least partially solubilizing the teicoplanin starting material.

Examples of said inert organic solvents are organic amides, alkyl ethers, ethers of glycols and polyols, phosphoramides, sulfoxides and aromatic compounds. Preferred examples of inert organic solvents are: dimethylformamide, dimethoxyethane, hexamethylphosphoramide, dimethylsulfoxide, benzene, toluene and mixtures thereof.

More preferably, this solvent is selected from acetonitrile, dimethylsulfoxyde, dimethylformamide. The formation of the activated ester is generally conducted in the presence of a base which does not interfere with the reaction course such as a trialkylamine like triethylamine, sodium or potassium carbonate or bicarbonate. Generally, the base is employed in a 2 to 6 molar proportion to the teicoplanin starting material and preferably it is used in about a three-fold molar excess. A preferred base is triethylamine.

The activated ester forming reagent is used in a large excess over the teicoplanin starting material. It is in general used in a 5 to 30 molar proportion and preferably, it is used in about a 20-times molar excess. The reaction temperature is between 10 °C and 80 °C and preferably is about 50 °C. As usual, the reaction time depends on the other specific reaction parameters and may be generally between 3 and 48h.

In this case, the reaction course may be followed by HPLC or TLC to determine when the reaction may be considered as completed and the procedures to recover the desired intermediate can be started. In general, it is isolated by precipitation with non-solvents or extraction with solvents and in general it is used as such in the next reaction step. If desired, however, it may be purified by a column chromatography such as flash column chromatography or reverse phase column chromatography.

The obtained "activated" ester intermediate is then reacted with a molar excess of the amine derivative of formula $NHR^1R^2$, preferably in the form of the corresponding acetate, in the presence of an organic polar solvent at a temperature between 5 °C and 60 °C.

The organic polar solvent can be in this case a polar protic solvent or an aprotic one.

Preferred examples of organic polar protic solvents are lower($C_2$-$C_4$) alkanols such as, ethanol, n-propanol, iso-propanol, n-butanol and the like, or mixtures thereof, preferably used in the dry form.

Preferred examples of organic polar aprotic solvent are N,N-dimethyformamide (DMF), hexamethylphosphoramide (HMPA), or mixtures thereof, 1, 3-dimethyl-3,4,5,6-tetrahydro-2(1H)pyrimidone (DMPU), dimethylsulfoxyde or dimethoxyethane.

The activated ester intermediate can be directly used in the reaction medium but it is preferably extracted before reacting with the molar excess of the amine.

The reaction can be carried out between 5 °C and 60 °C but the preferred temperature is generally comprised between 15 and 40 °C; more preferably at room temperature (20 °-25 °C), while a preferred molar proportion between the intermediate and the amine derivative as above defined is from 1:5 to 1:40, and most preferably is about 1 to 10

The reaction course may be monitored as usual and the reaction product may be isolated according to known per se techniques.

In the case of the compounds of formula I, wherein R and/or $R^6$ are different from hydrogen and the other substituents are as defined, a preferred process comprises starting from a $N^{15}$-alkyl or a $N^{15}$, $N^{15}$-dialkyl teicoplanin compound of the formula II above wherein R and $R^6$ are different from hydrogen,

protected on the 15-amino function, and reacting it with an activated ester forming reagent to give an "activated ester function" which can be easily substituted by a amino of formula $NHR^1R^2$ to give the corresponding amide.

The reaction conditions for the preparation of the above mentioned compounds of formula I are substantially the same conditions stated above. The protection of the $N^{15}$ function will depend on the alkyl substituent linked to the $N^{15}$ moiety. If R and $R^6$ are both different from hydrogen the protection is not necessary. If one of R and $R^6$ is hydrogen and the other is a bulky group included in its definition such as benzyl, p-nitrobenzyl and the like, generally it is not necessary to protect the $N^{15}$-amino function since its reactivity in the further reaction steps is substantially reduced or impaired so that no major undesired interferences are found. On the basis of the present disclosure and of the common general knowledge in the art, the skilled man is able to determine, without made experimentation, if a protection is needed for the $N^{15}$-amino group. The $N^{15}$-monoalkyl and the $N^{15}$, $N^{15}$-dialkyl teicoplanin compounds used as starting material can be prepared according to many procedures.

Methods for preparing starting compounds of this type are described in European Pat. Appln. Publication No. 276740 and in the European Patent Application Serial No. 89112587 and European Patent Application Serial No. 89111730 and European Patent Application Serial No. 89112590.

For example according to a preferred embodiment for preparing the $N^{15}$-mono alkyl derivative used as starting material, a compound of formula II wherein R and $R^6$ are hydrogen is reacted with an alkyl halide R-(halo) or $R^6$-(halo), wherein the alkyl portion is represented by an "alkyl" group which corresponds to the desired meaning of R or $R^6$ in the final product and the halide is preferably a chloride, bromide or iodide and most preferably bromide or chloride.

This reaction occurs at about room temperature in the presence of an organic or inorganic base of medium strength such as triethylamine, trimethylamine, tri-butylamine, sodium or potassium bicarbonate and the like.

Preferably, the reaction medium includes also an organic solvent capable of at least partially solubilizing the starting materials such as organic amides, alkyl ethers, ethers of glycols and polyols, phosphoramides, sulfoxides and aromatic compounds e.g., dimethylformamide, dimethoxyethane, hexamethylphosphoramlde, dimethylsulfoxide, benzene, toluene and mixtures thereof.

This solvent should have a low water content (generally below about 5%).

The alkyl halide and the starting teicoplanin compound derivative are preferably employed in about equimolecular proportion or in a slight molar excess of the alkyl halide so that the mono-alkyl derivative is obtained in good yields.

Another procedure for preparing a $N^{15}$-mono alkyl derivative falling within formula II comprises:

1a) adding to a suspension of a teicoplanin compound or a compound of formula II, wherein R and $R^6$ are hydrogen atoms in an organic polar protic solvent a suitable amount of an alkali metal borohydride at a temperature comprised between 0 °C and 30 °C

1b) adding thereto a molar excess of a carbonyl compound of at least 20 times with respect to teicoplanin starting material, and

1c) reacting the resulting solution with a reducing agent, preferably a metal alkali borohydride.

In this case among the organic polar protic solvents lower alkanols having from 1 to 4 carbon atoms are preferred. In particular, methanol is the preferred solvent.

Sometimes, in particular cases, a small amount of a polar aprotic co-solvent can be added to completely dissolve teicoplanin starting material during the course of the reaction, e.g. N,N-dimethylformamide, 1,3-dimethyl-3,4,5,6-tetrahydro-2(1H)-pyrimidinone (DMPU), dimethylsulfoxide.

The temperature at which the reaction is carried out is comprised between 0 °C and 30 °C, preferably it ranges from 10 °C to 15 °C.

It is important to use a large molar excess of carbonyl compound, generally from 20 to 100 times with respect to teicoplanin starting material. The carbonyl compound used has the same carbon skeleton of substituent $R^6$ (or R)

For example, if

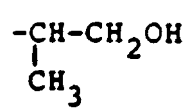

is the desired $N^{15}$ monoalkyl group $R^6$,
then

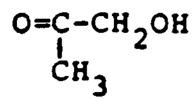

is the useful carbonyl compound which has to be employed as starting reactant.

Sodium borohydride is the preferred alkali metal borohydride used in step 1a).

As metal alkali borohydride used in the step 1c) the sodium borohydride is the preferred one, but also the potassium and the sodium cyano-borohydride can be used.

According to a preferred embodiment of this particular procedure it is important to maintain the reactants addition order as indicated above in steps 1a), 1b) and 1c).

In a further preferred embodiment of this procedure, before adding the excess of carbonyl compound to the reaction mixture, the alkali metal borohydride is allowed to completely decompose under stirring. Generally it takes a time comprised between 30 minutes and 120 minutes.

In particular, the carbonyl compound can be preferably added when the reaction mixture is a clear solution.

The suitable amount of alkali metal borohydride added can be different depending on the starting material, on the solvent used and on the temperature of the reaction, but it is advisable to add an amount of alkali metal borohydride such that the pH of the reaction mixture is alkaline, preferably between pH 8 and 10, as determined after diluting a sample of the non-aqueous reaction mixture with 4 volumes of water.

The reaction times of this procedure depend on the factors reported above such as the starting compound used, the solvent, the amount of alkali metal borohydride used and the like, and on the structure of reactant carbonyl compounds but, in general, it is preferred to leave the reaction mixture under stirring from 1 to 10 h after the addition of the reactants and before treating with the reducing agent. Said procedure is reported in the already cited European Patent Application Serial No. 89112587.

Another procedure, which permits obtaining the $N^{15}$, $N^{15}$-dialkyl teicoplanin compounds, see European Patent Application Serial No. 89112590, comprises adding to a suspension of a monoalkyl derivative of teicoplanin, in an organic polar protic solvent a molar excess of a carbonyl compound having the same carbon skeleton of the substituents R above, said molar excess being of at least 30 times with respect to the teicoplanin starting material, in the presence of an organic acid of such strength and in such amount that a sample of the obtained mixture diluted with four volumes of water shows a pH between 2.5 and 4, said acid being further characterized in that, under the reaction conditions, it cannot yield a reduction product which is a competitor of the carbonyl compound reactant in the reaction with the $N^{15}$ amino group of the teicoplanin moiety, and reacting the resulting mixture with a metal alkali borohydride at a temperature between 0 °C and 60 °C.

Among the organic polar protic solvents, alkyl alcohols having from 1 to 4 carbon atoms are preferred. Examples of alcohols which can be used are methanol, ethanol, propanol and butanol. In particular, methanol is preferred.

Sometimes in particular cases, a small amount of a polar co-solvent can be added to completely dissolve teicoplanin starting material during the course of the reaction, e.g. N,N-dimethylformamide, 1,3-dimethyl-3,4,5,6-tetrahydro-2(1H)-pyrimidinone (DMPU), dimethylsulfoxide.

As the reducing agents, alkali metal borohydrides are used, among which sodium borohydride is the preferred one, but also potassium and sodium cyanoborohydrides can be conveniently used.

The temperature can be preferably maintained between 30 °C and 40 °C.

The molar excess of the reactants with respect to teicoplanin ranges preferably from 30 to 300 times for aldehydes or ketones.

The organic acid which is used is characterized by having such a strength to show a pH between 2.5 and 4 when a sample of the reaction mixture is added to 4 volumes of water before adding the reducing agent. Furthermore, said acid must not negatively interfere in the reaction course. It means that it preferably has not to be reduced by the reducing agent employed ($NaBH_4$ and the like) or, if a reaction occurs, the product of this reaction is not a competitor with the carbonyl compound choosen as starting material.

The term "competitor" as used in specification means any group which can be linked to an amino group with a bond like a "Schiff base" and subsequently reduced to an alkyl moiety, being said group different from the carbonyl compound used as starting material. Thus, in a preferred embodiment of the present invention when an aldehyde is used as the carbonyl compound the suitable acid to be preferably

used is the carboxylic acid corresponding to said aldehyde; for example, formic acid with formaldehyde, acetic acid with acetaldehyde and so on. In the case that a keto compound is used as the starting material, a preferred acid can be choosen among a ($C_1$-$C_6$)alkyl sulfonic acid or a ($C_6$-$C_{10}$)aryl sulfonic acid such as for example paratoluensulfonic acid, methanesulfonic acid and the like. The amount of acid used depends on several factors such as the teicoplanin starting material and the carbonyl compound but usually the desired pH is obtained when a molar excess of acid is used from 5 to 20 times with respect to teicoplanin.

The following table (Table I) reports the structure formulas of representative examples of compounds that can be prepared according to the process of the invention:

**TABLE I**

| Compound | A | B | M | R | $R^6$ | $Y=NR^1R^2$ |
|---|---|---|---|---|---|---|
| 1 | GA | GAC | M | H | H | $NH_2$ |
| 2 | H | H | H | do | do | do |
| 3 | GA | GAC | M | do | do | $NHCH_3$ |
| 4 | H | H | H | do | do | do |
| 5 | GA | GAC | M | do | do | $N(CH_3)_2$ |

GA stands for N-[($C_9$-$C_{11}$)aliphatic acyl]-beta-D-2-deoxy-2-aminoglucopyranosyl

$GA_{(2)}$ stands for N-(8-methylnonanoyl)-beta-D-2-deoxy-2-aminoglucopyranosyl

$GA_{(2-3)}$ stands for N-(8-methylnonanoyl)-beta-D-2-deoxy-2-aminoglucopyranosyl plus N-(decanoyl)-beta-D-2-deoxy-2-aminoglucopyranosyl

GAC stands for N-acetyl-beta-D-2-deoxy-2-aminoglucopyranosyl

M stands for alpha-D-mannopyranosyl

H stands for a hydrogen atom

Continued.......

**TABLE I**

| Compound | A | B | M | R | $R^6$ | $Y=NR^1R^2$ |
|---|---|---|---|---|---|---|
| 6 | GA | GAC | M | H | H | $NH(CH_2)_2NH_2$ |
| 7 | H | H | H | do | do | $NH(CH_2)_2\ NH_2$ |
| 8 | GA | GAC | M | do | do | $NH(CH_2)_3\ NH_2$ |
| 9 | H | H | H | do | do | do |
| 10 | GA | GAC | M | do | do | $NH(CH_2)_4\ NH_2$ |
| 11 | H | H | H | do | do | do |
| 12 | GA | GAC | M | do | do | $NH(CH_2)_6\ NH_2$ |
| 13 | H | H | H | do | do | do |

Continued.......

**TABLE I**

| Compound | A | B | M | R | $R^6$ | $Y=NR^1R^2$ |
|---|---|---|---|---|---|---|
| 14 | GA | GAC | M | H | H | $NH(CH_2)_2$ $NHCH_3$ |
| 15 | H | H | H | do | do | do |
| 16 | GA | GAC | M | do | do | $NH(CH_2)_3$ $NHCH_3$ |
| 17 | H | H | H | do | do | do |
| 18 | GA | GAC | M | do | do | $NH(CH_2)_2$ $NHC_2H_5$ |
| 19 | H | H | H | do | do | do |
| 20 | GA | GAC | M | do | do | $NH(CH_2)_3$ $NHC_2H_5$ |

Continued.......

TABLE I

| Compound | A | B | M | R | $R^6$ | $Y=NR^1R^2$ |
|---|---|---|---|---|---|---|
| 21 | GA | GAC | M | H | H | $NH(CH_2)_2\ N(CH_3)_2$ |
| 22 | H | H | H | do | do | do |
| 23 | GA | GAC | M | do | do | $NH(CH_2)_3\ N(CH_3)_2$ |
| 24 | H | H | H | do | do | do |
| 25 | GA | GAC | M | do | do | $NH(CH_2)_4\ N(CH_3)_2$ |
| 26 | H | H | H | do | do | do |

Continued.......

TABLE I

| Compound | A | B | M | R | $R^6$ | $Y=NR^1R^2$ |
|---|---|---|---|---|---|---|
| 27 | GA | GAC | M | H | H | $NH(CH_2)_5\ N(CH_3)_2$ |
| 28 | H | H | H | do | do | do |
| 29 | GA | GAC | M | do | do | $NH(CH_2)_7\ N(CH_3)_2$ |
| 30 | H | H | H | do | do | do |
| 31 | GA | GAC | M | do | do | $NH(CH_2)_3\ N(C_2H_5)_2$ |
| 32 | $GA_{(2)}$ | do | do | do | do | do |
| 33 | H | H | H | do | do | do |

Continued.......

**TABLE I**

| Compound | A | B | M | R | $R^6$ | $Y=NR^1R^2$ |
|---|---|---|---|---|---|---|
| 34 | GA | GAC | M | H | H | $NH(CH_2)_3N(n-C_4H_9)_2$ |
| 35 | H | H | H | do | do | do |
| 36 | GA | GAC | M | do | do | $N(CH_3)(CH_2)_2NHCH_3$ |
| 37 | H | H | H | do | do | do |
| 38 | GA | GAC | M | do | do | $N(CH_3)(CH_2)_3NHCH_3$ |
| 39 | $GA_{(2-3)}$ | do | do | do | do | do |
| 40 | H | H | H | do | do | do |
| 41 | GA | GAC | M | do | do | $N(CH_3)(CH_2)_2N(CH_3)_2$ |

Continued.......

**TABLE I**

| Compound | A | B | M | R | $R^6$ | $Y=NR^1R^2$ |
|---|---|---|---|---|---|---|
| 42 | H | H | H | H | H | $N(CH_3)(CH_2)_3N(CH_3)_2$ |
| 43 | GA | GAC | M | do | do | 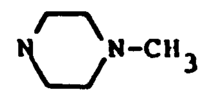 |
| 44 | H | H | M | do | do | do |
| 45 | GA | GAC | M | do | do | 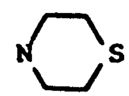 |
| 46 | H | H | H | do | do | 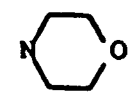 |
| 47 | GA | GAC | M | do | do | $NHCH(COOCH_3)(CH_2)_4NH_2$ |
| 48 | $GA_{(2-3)}$ | do | do | do | do | do |
| 49 | H | H | H | do | do | do |

Continued.......

## TABLE I

| Compound | A | B | M | R | $R^6$ | $Y=NR^1R^2$ |
|---|---|---|---|---|---|---|
| 50 | GA | GAC | M | H | H | $NH\ CH\ (COOH)(CH_2)_4\ NH_2$ |
| 51 | H | H | H | do | do | do |
| 52 | GA | GAC | M | do | do | $NH(CH_2)_3\ NH(CH_2)_2\ OH$ |
| 53 | $GA_{(2-3)}$ | do | do | do | do | do |
| 54 | H | H | H | do | do | do |
| 55 | $GA_{(2)}$ | GAC | M | do | do | $NH(CH_2)_3-N(CH_3)_2$ |

Continued.......

TABLE I

| Compound | A | B | M | R | $R^6$ | $Y=NR^1R^2$ |
|---|---|---|---|---|---|---|
| 56* | $GA_{(2)}$ | GAC | M | H | H | $NH(CH_2)_3-N(CH_3)_2$ |
| 57* | do | do | do | do | do | do |
| 58* | do | do | do | do | do | do |
| 59 | H | H | H | do | $CH(CH_3)CH_2OH$ | $NH(CH_2)_3-N(CH_3)_2$ |
| 60 | GA | GAC | M | do | $CH_2CH_2-N(CH_3)_2$ | $NH(CH_2)_2\ NH_2$ |

* The compounds 56, 57 and 58 are characterized by having a different salt form which is HCl, 2HCl and AcOH respectively.

Continued.......

**TABLE I**

| Compound | A | B | M | R | $R^6$ | $Y=NR^1R^2$ |
|---|---|---|---|---|---|---|
| 61 | H | H | H | H | $CH_3$ | $NH(CH_2)_3NH_2$ |
| 62 | GA | GAC | M | do | $CH(CH_3)CH_2OH$ | do |
| 63 | H | H | H | do | do | do |
| 64 | GA | GAC | M | do | do | $NH(CH_2)_3N(CH_3)_2$ |
| 65 | do | do | do | do | $CH(CH_3)CH(CH_3)OH$ | do |
| 66 | H | H | H | do | do | do |

According to the process of the present invention described above, two preferred methods for obtaining the amides of formula I are shown below. The first method (procedure A) comprises :

1) the reaction between teicoplanin $A_2$ complex and benzylchloroformate in order to obtain the protected $N^{15}$-benzyloxycarbonyl teicoplanin $A_2$ complex ($N^{15}$-CBZ teicoplanin $A_2$ complex.

2) The reaction between said protected compound and chloroacetonitrile in order to obtain the activate cyanomethyl ester.

3) The reaction between the $N^{15}$ CBZ teicoplanin $A_2$ cyanomethyl ester and the proper amine (as reported in Table II)

4) The preparation of the $N^{63}$ carboxyamide and its deprotection.

5) The purification of the products by reverse phase column chromatography.

The second method (procedure B) comprises:

1) Thee reaction between deglucoteicoplanin and tert-butyl-2,4,5-trichlorophenyl carbonate in order to prepare $N^{15}$-tert-butyloxy carbonyl deglucoteicoplanin (t-BOC).

2) The reaction between said protected compound and chloroacetonitrile in order to obtain the activated cyanomethyl ester.

3) The reaction between the $N^{15}$-TBOC deglucoteicoplanin, cyanomethyl ester and the proper amine (reported in Table II)

4) The preparation of the $N^{63}$ carboxyamide and its deprotection.

5) The purification of the products by reverse phase column chromatography.

The yields given in Table II refer to the compounds obtained according to the general methods above summarized (procedures A and B) and are molar yields. However some of the compounds listed in Table I are also obtained with different yields according to modified procedures.

In these cases representative examples of preparation of each compound according to such alternative methods are described in the experimental section. All amides of teicoplanin $A_2$ complex listed in the Table II are free from the component 1, which is transformed in the component 3 during the hydrogenolysis of the $N^{15}$-CBZ protecting group. When alternative protecting groups of the amino function at C-15 are used which are removable under neutral or controlled mild acidic conditions, the amides obtained are derivatives of components 1 to 5 of the complex.

Table II reports for each compound the method of preparation (procedure A or B), the starting material (i.e. the proper amine) the yield and the salt form obtained.

Legenda:

AcOH stands for acetate
HCl stands for hydrochloride
TFA stands for trifluoroacetate
FB stands for free bases
TEICO stands for Teicoplanin $A_2$
DEGLU stands for deglucoteicoplanin $A_2$
TEICO $A_{2,2}$ stands for Teicoplanin $A_2$ component 2
TEICO $A_{2,2-3}$ stands for Teicoplanin $A_2$ components 2, 3

TABLE II

| Compound | Method of Preparation (procedure) | Starting material | Yields % | Salt form |
|---|---|---|---|---|
| 1 | A | TEICO $NH_3$ | 49 | HCl |
| 2 | B | DEGLU do | 41 | TFA |
| 3 | A | TEICO $NH_2CH_3$ | 58 | HCl |
| 4 | B | DEGLU do | 40 | TFA |
| 5 | A | TEICO $NH(CH_3)_2$ | 87 | FB |
| 6 | A | TEICO $NH_2(CH_2)_2NH_2$ | 76 | AcOH |
| 7 | B | DEGLU do | 54 | 2 HCl |
| 8 | A | TEICO $NH_2(CH_2)_3NH_2$ | 84 | AcOH |
| 9 | B | DEGLU do | 61 | 2 TFA |

Continued.....

TABLE II

| Compound | Method of Preparation (procedure) | Starting material | | Yields % | Salt form |
|---|---|---|---|---|---|
| 10 | A | TEICO | $NH_2(CH_2)_4NH_2$ | 81 | AcOH |
| 11 | B | DEGLU | do | 66 | 2 HCl |
| 12 | A | TEICO | $NH_2(CH_2)_6NH_2$ | 78 | AcOH |
| 13 | B | DEGLU | do | 64 | 2 HCl |
| 14 | A | TEICO | $NH_2(CH_2)_2NHCH_3$ | 68 | AcOH |
| 15 | B | DEGLU | do | 67 | 2 TFA |
| 16 | A | TEICO | $NH_2(CH_2)_3NHCH_3$ | 79 | AcOH |
| 17 | B | DEGLU | do | 79 | 2 HCl |

Continued....

TABLE II

| Compound | Method of Preparation (procedure) | Starting material | | Yields % | Salt form |
|---|---|---|---|---|---|
| 18 | A | TEICO | $NH_2(CH_2)_2NHC_2H_5$ | 66 | AcOH |
| 19 | B | DEGLU | do | 70 | 2 HCl |
| 20 | A | TEICO | $NH_2(CH_2)_3NHC_2H_5$ | 85 | AcOH |
| 21 | A | TEICO | $NH_2(CH_2)_2\ N(CH_3)_2$ | 81 | AcOH |
| 22 | B | DEGLU | do | 63 | 2 TFA |
| 23 | A | TEICO | $NH_2(CH_2)_3\ N(CH_3)_2$ | 85 | AcOH |
| 24 | B | DEGLU | do | 66 | HCl |

Continued....

TABLE II

| Compound | Method of Preparation (procedure) | Starting material | Yields % | Salt form |
|---|---|---|---|---|
| 25 | A | TEICO $NH_2(CH_2)_4N(CH_3)_2$ | 86 | AcOH |
| 26 | A | DEGLU do | 57 | HCl |
| 27 | A | TEICO $NH_2(CH_2)_5N(CH_3)_2$ | 82 | AcOH |
| 28 | B | DEGLU do | 68 | 2 HCl |
| 29 | A | TEICO $NH_2(CH_2)_7N(CH_3)_2$ | 79 | HCl |
| 30 | B | DEGLU do | 64 | 2 TFA |
| 31 | A | TEICO $NH_2(CH_2)_3N(C_2H_5)_2$ | 83 | 2 HCl |
| 32 | A | TEICO $A_{2,2}$ do | 80 | HCl |

Continued....

TABLE II

| Compound | Method of Preparation (procedure) | Starting material | Yields % | Salt form |
|---|---|---|---|---|
| 33 | B | DEGLU do | 63 | 2 HCl |
| 34 | A | TEICO $NH_2(CH_2)_3N(n-C_4H_9)_2$ | 71 | AcOH |
| 35 | B | DEGLU do | 53 | HCl |
| 36 | A | TEICO $NH(CH_3)(CH_2)_2NHCH_3$ | 77 | AcOH |
| 37 | B | DEGLU do | 72 | TFA |
| 38 | A | TEICO $NH(CH_3)(CH_2)_3NHCH_3$ | 87 | AcOH |
| 39 | A | TEICO $A_{2'2-3}$ do | 86 | AcOH |
| 40 | B | DEGLU do | 74 | 2 HCl |

Continued....

TABLE II

| Compound | Method of Preparation (procedure) | Starting material | Yields % | Salt form |
|---|---|---|---|---|
| 41 | A | TEICO $NH(CH_3)(CH_2)_2N(CH_3)_2$ | 78 | HCl |
| 42 | B | DEGLU do | 51 | HCl |
| 43 | A | TEICO 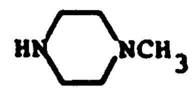 | 82 | HCl |
| 44 | B | DEGLU do | 46 | 2TFA |
| 45 | A | TEICO 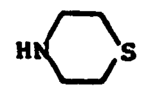 | 82 | F.B. |
| 46 | B | DEGLU 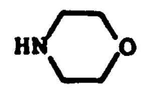 | 55 | F.B. |
| 47 | A | TEICO $NH_2$-$CH(COOCH_3)(CH_2)_4NH_2$ | 61 | AcOH |
| 48 | A | TEICO $A_{2'2-3}$ do | 63 | AcOH |

Continued....

TABLE II

| Compound | Method of Preparation (procedure) | Starting material | | Yields % | Salt form |
|---|---|---|---|---|---|
| 49 | B | DEGLU | do | 66 | 2 HCl |
| 50 | * | Compound 47 | $NH_2CH(COOH)(CH_2)_4NH_2$ | 64 | F.B. |
| 51 | * | Compound 49 | do | 53 | HCl |
| 52 | A | TEICO | $NH_2(CH_2)_3-NH(CH_2)_2OH$ | 87 | F.B. |
| 53 | A | TEICO $A_{2,2-3}$ | do | 89 | AcOH |
| 54 | B | DEGLU | do | 71 | HCl |

* According to the saponification procedure described in the European Patent Application Publication No. 218099.

Continued....

TABLE II

| Compound | Method of Preparation (procedure) | Starting material | Yields % | Salt form |
|---|---|---|---|---|
| 55 | Ex. 5 | Compound 56 | 86 | - |
| 56 | A | TEICO $A_{2,2}$ $NH_2(CH_2)_3-N(CH_3)_2$ | 96 | HCl |
| 57 | Ex. 5 | Compound 58 | 95 | 2 HCl |
| 58 | Ex. 5 | Compound 56 | 89 | AcOH |
| 59 | A | * $NH_2(CH_2)_3-N(CH_3)_2$ | 77 | - |
| 60 | A | * $NH_2(CH_2)_2$ $NH_2$ | 61 | - |

* See the next page

Continued....

TABLE II

| Compound | Method of Preparation (procedure) | | Starting material | Yields % | Salt form |
|---|---|---|---|---|---|
| 61 | B | * | $NH_2(CH_2)_3\ NH_2$ | 71 | - |
| 62 | A | * | do | 58 | - |
| 63 | B | * | do | 52 | - |
| 64 | A | * | $NH_2(CH_2)_3-N(CH_3)_2$ | - | - |
| 65 | A | * | do | - | - |
| 66 | B | * | do | - | - |

* Starting from the $N^{15}$-alkyl derivatives of Teicoplanin. See the specific examples.

Table III reports $^1H$ NMR data obtained at 250 MHz with a Bruker AM-250 Spectrometer in DMSO-$d_6$ at 20 °C, at a sample concentration of 20 mg/ml (internal standard: TMS, = 0.00 ppm).

## TABLE III

$^{1}H$-NMR Spectra (delta, ppm) in DMSO-$d_6$

| Compound | |
|---|---|
| 1 | 2.02, 1.38, 1.16, 0.83 (acyl chain), 1.87 (acetylglucosamine), 3.49 (mannose); 4.11-5.62 (peptidic CH's); 6.18-8.63 (aromatic protons and peptidic NH's) |
| 2 | 4.10-5.80 (peptidic CH's); 6.31-8.33 (aromatic protons and peptidic NH's) |
| 3 | 2.62 ($N-CH_3$); 2.02-1.38, 1.17, 0.84 (acyl chain); 1.86 (acetylglucosamine); 3.48 (mannose); 4.10-5.62 (peptidic CH's); 6.15-8.76 (aromatic protons and peptidic NH's) |
| 4 | 2.66 ($N-CH_3$); 4.12-5.65 (peptidic CH's); 6.18-8.51 (aromatic protons and peptidic NH's) |

Continued....

## TABLE III

$^1$H-NMR Spectra (delta ppm) in DMSO-$d_6$

| Compound | |
|---|---|
| 6 | 2.62 ($CH_2$), 2.01, 1.39, 1.15, 0.82 (acyl chain); 1.86 (acetylglucosamine); 3.47 (mannose); 4.13-5.62 (peptidic CH's); 6.29-8.48 (aromatic protons and peptidic NH's) |
| 7 | 2.92 ($CH_2$); 4.14-5.49 (peptidic CH's); 6.18-8.80 (aromatic protons and peptidic NH's) |
| 8 | 3.02, 2.81 ($CH_2$); 2.01, 1.38, 1.17, 0.83 (acyl chain); 1.87 (acetyl-glucosamine); 3.48 (mannose); 4.12-5.63 (peptidic CH's); 6.18-8.57 (aromatic protons and peptidic NH's) |
| 9 | 2.78, 1.77 ($CH_2$); 4.12-5.63 (peptidic CH's); 6.18-8.79 (aromatic protons and peptidic NH's) |

Continued....

## TABLE III

$^{1}H$-NMR Spectra (delta ppm) in DMSO-$d_6$

| Compound | |
|---|---|
| 11 | 2.77, 1.52 ($CH_2$), 4.13-5.63 (peptidic CH's); 6.19-8.77 (aromatic protons and peptidic NH's) |
| 13 | 2.76, 1.49 ($CH_2$); 4.12-5.62 (peptidic CH's); 6.19-8.62 (aromatic protons and peptidic NH's) |
| 14 | 2.52 (N-$CH_3$), 3.03 ($CH_2$); 2.03, 1.45, 1.18, 0.84 (acyl chain); 1.89 (acetylglucosamin); 3.47 (mannose); 4.09-5.63 (peptidic CH's); 6.32-7.80 (aromatic protons) |
| 15 | 2.61 (N-$CH_3$); 3.47, 3.02 ($CH_2$); 4.15-5.62 (peptidic CH's); 6.19-8.37 (aromatic protons and peptidic NH's) |

Continued....

## TABLE III

$^1$H-NMR Spectra (delta ppm) in DMSO-$d_6$

| Compound | |
|---|---|
| 16 | 2.53 ($N-CH_3$); 3.03, 2.72 ($CH_2$); 1.99, 1.45, 1.14, 0.83 (acyl chain); 1.88 (acetylglucosamine); 3.48 (mannose); 4.11-5.67 (peptidic CH's); 6.30-8.62 (aromatic protons and peptidic NH's) |
| 17 | 2.53 ($N-CH_3$); 3.09, 1.77 ($CH_2$); 4.14-5.81 (peptidic CH's), 6.19-8.83 (aromatic protons and peptidic NH's) |
| 18 | 1.05, 3.48 ($N-C_2H_5$); 2.01, 1.45, 1.20, 0.82 (acyl chain); 1.86 (acetylglucosamine); 3.48 (mannose); 4.12-5.63 (peptidic CH's); 6.31-8.72 (aromatic protons and peptidic NH's) |
| 19 | 1.18, 3.48 ($N-C_2H_5$); 3.47, 3.01 ($CH_2$); 4.16-5.61 (peptidic CH's), 6.20-8.37 (aromatic protons and peptidic CH's) |

Continued....

TABLE III

$^1$H-NMR Spectra (delta ppm) in DMSO-$d_6$

| Compound | |
|---|---|
| 20 | 1.19, 3.01 (N-$C_2H_5$); 2.02, 1.48, 1.15, 0.84 (acyl chain); 1.87 (acetylglucosamine); 3.49 (mannose); 4.11-5.62 (peptidic CH's); 6.29-8.59 (aromatic protons and peptidic NH's) |
| 26 | 2.68, 2.68 (N-$CH_3$); 2.92, 1.54 (aliphatic side chain); 4.12-5.63 (peptidic CH's); 6.64-8.62 (aromatic protons and peptidic NH's) |
| 28 | 2.71, 2.70 (N-$CH_3$); 1.61, 1.48, 1.29 (aliphatic side chain); 4.09-5.63 (peptidic CH's), 6.21-8.61 (aromatic protons and peptidic NH's) |
| 30 | 2.71, 2.69 (N-$CH_3$); 2.97, 1.60, 1.41, 1.22 (aliphatic side chain); 4.08-5.54 (peptidic CH's); 6.09-8.58 (aromatic protons and peptidic NH's) |

Continued....

## TABLE III

$^1$H-NMR Spectra (delta ppm) in DMSO-$d_6$

| Compound | |
|---|---|
| 36 | 2.54, 2.52 ($N-CH_3$); 3.02 ($CH_2$); 2.02, 1.46, 1.18, 0.83 (acyl chain); 1.89, (acetylglucosamine); 4.12-5.62 (peptidic CH's); 6.32-8.54 (aromatic protons and peptidic NH's) |
| 37 | 2.56 ($N-CH_3$), 2.91, 2.86 ($CH_2$); 4.13-5.62 (peptidic CH's); 6.19-8.52 (aromatic protons and peptidic NH's) |
| 38 | 2.51 ($N-CH_3$); 2,44, 2.85 ($CH_2$); 2.02, 1.43, 1.17, 0.85 (acyl chain); 1.87 (acetylglucosamine); 3.47 (mannose); 4.08-5.69 (peptidic CH's); 6.27-8.62 (aromatic protons and peptidic NH's) |
| 40 | 2.71 ($N-CH_3$); 2.44, 1.80, ($CH_2$); 4.15-5.63 (peptidic CH's); 6.19-8.45 (aromatic protons and peptidic NH's) |

Continued....

TABLE III

| Compound | $^1$H-NMR Spectra (delta ppm) in DMSO-$d_6$ |
|---|---|
| 42 | 2.86, 2.76 ($N-CH_3$); 1.48 ($CH_2$); 4.15-5.75 (peptidic CH's); 6.24-8.75 (aromatic protons and peptidic NH's) |
| 52 | 3.03, 2.64, 2.05 ($CH_2$-side chain); 0.83, 1.15, 1.45, 2.02 (acyl chain); 1.85 (acetylglucosamin); 4.13-5.70 (peptidic CH's); 6.31-8.63 (aromatic protons and peptidic NH's) |
| 53 | 3.05, 2.63, 2.06 ($CH_2$-side chain); 0.83, 1.13, 1.46, 2.02 (acyl chain); 1.87 (acetylglucosamin); 3.51 (mannose), 4.14-5.65 (peptidic CH's); 6.31-8.56, (aromatic protons and peptidic NH's) |
| 55 | 2.09 ($N-CH_3$); 2.14, 3.12 ($CH_2$-side chain); 0.82, 1.15, 1.46, 2.03 (acyl chain), 1.87, (acetylglucosamin); 4.13-5.64 (peptidic CH's); 6.31-8.54 (aromatic protons and peptidic NH's) |

The antibacterial acitivity of the compounds obtained with the process of the present invention can be demonstrated in vitro by means of standard agar-dilution tests.

Isosensitest broth (Oxoid) and Todd-Hewitt broth (Difco) are used for growing staphylococci and streptococci, respectively. Broth cultures are diluted so that the final inoculum is about $10^4$ colony forming units/ml (CFU/ml). Minimal inhibitory concentration (MIC) is considered as the lowest concentration which shows no visible growth after 18-24 h incubation at 37°C. The results of the antibacterial testing of

representative compounds of Formula I are summarized in Table IV below:

**TABLE IV**

*In vitro* (MIC microgram/ml)

| | Compounds No. | | | | | |
|---|---|---|---|---|---|---|
| | 1 | 2 | 3 | 4 | 6 | 7 |
| S. haemolyticus 602 | 1 | 0.12 | 1 | 0.12 | 0.12 | 0.12 |
| S. aureus tour ($10^4$ cfu/ml) | 0.12 | 0.06 | 0.12 | 0.06 | 0.12 | 0.06 |
| S. aureus tour (L 165) + 30% bovine serum | 1 | 0.12 | 1 | 0.25 | 1 | 0.25 |
| S. epidermidis ATCC 12228 | 0.06 | 0.03 | 0.12 | 0.03 | 0.06 | 0.03 |
| S. pyogenes C 203 | 0.06 | 0.06 | 0.06 | 0.06 | 0.06 | 0.06 |
| S. pneumoniae U C41 | 0.06 | 0.03 | 0.06 | 0.12 | 0.12 | 0.12 |
| S. faecalis ATCC 7080 | 0.12 | 0.12 | 0.12 | 0.12 | 0.12 | 0.12 |
| E. coli SKF 12140 | > 128 | 32 | > 128 | 32 | > 128 | 4 |
| P. vulgaris X 19H ATCC 881 | > 128 | 128 | > 128 | 128 | > 128 | 16 |
| P. aeruginosa ATCC 10145 | > 128 | 32 | > 128 | 64 | > 128 | 16 |

**TABLE IV**

*In vitro* (MIC microgram/ml)

| | Compounds No. | | | | | |
|---|---|---|---|---|---|---|
| | 8 | 9 | 11 | 13 | 14 | 15 |
| S. hemolyticus 602 | 0.12 | 0.12 | 0.12 | 0.12 | 0.12 | 0.06 |
| S. areus tour ($10^4$ cfu/ml) | 0.12 | 0.12 | 0.06 | 0.12 | 0.06 | |
| S. aureus tour (L 165) + 30 % bovine serum | 0.25 | 0.5 | 0.25 | 0.25 | 1 | 0.12 |
| S. epidermidis ATCC 12228 | 0.06 | 0.06 | 0.06 | 0.06 | 0.12 | 0.03 |
| S. pyogenes C 203 | 0.03 | 0.06 | 0.06 | 0.06 | 0.06 | 0.06 |
| S. pneumoniae U C41 | 0.12 | 0.12 | 0.12 | 0.06 | 0.12 | 0.12 |
| S. faecalis ATCC 7080 | 0.12 | 0.12 | 0.12 | 0.12 | 0.12 | 0.12 |
| E. coli SKF 12140 | > 128 | 4 | 8 | 4 | > 128 | 4 |
| P. vulgaris X 19H ATCC 881 | > 128 | 32 | 32 | 64 | > 128 | 32 |
| P. aeruginosa ATCC 10145 | > 128 | 32 | 32 | 64 | 128 | 32 |

Continued.....

TABLE IV

In vitro (MIC microgram/ml)

| | Compounds No. | | | | | |
|---|---|---|---|---|---|---|
| | 16 | 17 | 18 | 19 | 20 | 26 |
| S. haemolyticus 602 | 0.12 | 0.25 | 0.12 | 0.12 | 0.12 | 0.12 |
| S. aureus tour ($10^4$ cfu/ml) | 0.12 | 0.06 | 0.12 | 0.06 | 0.12 | 0.06 |
| S. aureus tour (L 165) + 30% bovine serum | 1 | 0.12 | 1 | 0.12 | 2 | 0.12 |
| S. epidermidis ATCC 12228 | 0.06 | 0.06 | 0.06 | 0.06 | 0.06 | 0.06 |
| S. pyogenes C 203 | 0.06 | 0.06 | 0.06 | 0.06 | 0.06 | 0.06 |
| S. pneumoniae U C41 | 0.12 | 0.12 | 0.12 | 0.12 | 0.12 | 0.12 |
| S. faecalis ATCC 7080 | 0.12 | 0.12 | 0.12 | 0.12 | 0.12 | 0.12 |
| E. coli SKF 12140 | > 128 | 4 | > 118 | 8 | > 128 | 8 |
| P. vulgaris X 19H ATCC 881 | > 128 | 16 | > 128 | 64 | > 128 | 32 |
| P. aeruginosa ATCC 10145 | > 128 | 16 | 128 | 32 | > 128 | 32 |

TABLE IV

*In vitro* (MIC microgram/ml)

| | Compounds No. | | | | | |
|---|---|---|---|---|---|---|
| | 28 | 30 | 36 | 37 | 40 | 42 |
| S. haemolyticus 602 | 0.12 | 0.12 | 1 | 0.12 | 0.06 | 0.06 |
| S. aureus tour ($10^4$ cfu/ml) | 0.06 | 0.06 | 0.12 | 0.12 | 0.06 | 0.06 |
| S. aureus tour (L 165) + 30% bovine serum | 0.25 | 0.5 | 1 | 1 | 0.12 | 0.12 |
| S. epidermidis ATCC 12228 | 0.06 | 0.06 | 0.06 | 0.06 | 0.06 | 0.06 |
| S. pyogenes C 203 | 0.06 | 0.06 | 0.06 | 0.12 | 0.06 | 0.06 |
| S. pneumoniae U C41 | 0.12 | 0.12 | 0.12 | 0.12 | 0.12 | 0.06 |
| S. faecalis ATCC 7080 | 0.12 | 0.12 | 0.12 | 0.12 | 0.12 | 0.12 |
| E. coli SKF 12140 | 8 | 16 | > 128 | 16 | 2 | 8 |
| P. vulgaris X 19H ATCC 881 | 128 | 128 | > 128 | > 128 | 32 | 64 |
| P. aeruginosa ATCC 10145 | 64 | 128 | > 128 | 64 | 16 | 64 |

The $ED_{50}$ values (mg/Kg) of representative compounds of the invention *in vivo* tests in mice experimentally infected with *S. pyogenes* L 49 according to the procedure described by V. Arioli et al., Journal of Antibiotics 29, 511 (1976) are reported in Table V below:

TABLE V

| Compound | $ED_{50}$ (mg/Kg) Route of administration | |
|---|---|---|
| | o s | s c |
| 1 | 139 | 0.08 |
| 2 | > 300 | 1.7 |
| 6 | 139 | 0.18 |
| 7 | N.T. | 1.25 |
| 8 | 89.6 | 0.08 |
| 9 | > 300 | 1.25 |
| 11 | > 300 | 0.54 |
| 13 | > 300 | 0.54 |
| 14 | 173 | 0.08 |
| 16 | 112 | 0.14 |
| 18 | about 300 | 0.18 |
| 20 | 112 | 0.10 |
| 28 | > 300 | 0.72 |
| 30 | > 300 | 0.95 |
| 36 | 72 | 0.1 |
| 40 | > 300 | 0.95 |
| 42 | > 300 | 1.25 |
| N.T. Not tested | | |

In view of the above reported antimicrobial activity, the compounds prepared by the process of the present invention can effectively be employed as the active ingredients of antimicrobial preparations used in human and veterinary medicine for the prevention and treatment of infectious diseases caused by pathogenic bacteria which are susceptible to said active ingredients.
In such treatments, these compounds may be employed as such or in the form of mixtures in any proportion. The compounds prepared according to the present invention can be administered orally, topically or parenterally wherein however, the parenteral administration is preferred. Depending on the route of administration, these compounds can be formulated into various dosage forms according to known per sé techniques.

The following examples further illustrate the invention and must not be construed as limiting it.

General Procedure A

1- Preparation of $N^{15}$-benzyloxycarbonyl (CBZ) teicoplanin $A_2$ complex.

A solution of 4.5 ml of benzyl chloroformate in 10 ml of dry acetone is added dropwise at room temperature to a stirred solution of 45 g (about 24 mmol) of teicoplanin $A_2$ complex and of 6 ml (about 44 mmol) of triethylamine (TEA) in 300 ml of dimethylformamide (DMF). After about 60 min, 600 ml of ethyl ether is added and the precipitate (about 59 g) is collected by filtration and re-dissolved in 2.5 L of a mixture acetone : water, 1:1 (v/v). The resulting solution is concentrated at 35 °C under reduced pressure to a volume of about 1.6 L, then it is extracted with 1.6 L of ethyl ether which is separated and discarded.

The aqueous layer is adjusted to pH 4.8 with glacial acetic acid and extracted with 1.5 L of n-butanol. The organic layer is separated, washed with 1.5 L of water (2 x 750 ml), then it is concentrated to a volume of about 200 ml at 45 °C under reduced pressure. On adding ethyl acetate (about 800 ml) a solid separates which is collected by filtration, washed with ethyl ether (about 500 ml) and dried at room temperature in vacuo overnight, yielding 45.7 g (about 96%) of pure title compound.

2 - Preparation of $N^{15}$-CBZ - teicoplanin $A_2$ complex, cyanomethyl ester.

To a stirred solution of 45 g (about 22 mmol) of $N^{15}$-CBZ-teicoplanin $A_2$ complex in 450 ml of DMF, 5.25 ml (about 37 mmol) of TEA and 60 ml of chloroacetonitrile are added at room temperature. After 20 h, the reaction mixture is poured into 4.5 L of ethyl acetate and the precipitate (about 50 g) is collected by filtration and re-dissolved in 900 ml of a mixture methanol : water 1:1 (v/v). The resulting solution is adjusted

to pH 5.5 with glacial acetic acid, then 1.1 L of n-butanol is added. Most of the methanol is evaporated at 35°C under reduced pressure to obtain a mixture (about 1.5 L) of n-butanol and water from which the organic layer is separated, washed with 500 ml of water and concentrated at 40°C under reduced pressure to a volume of about 200 ml. On adding 800 ml of ethyl acetate a solid separates which is collected, washed with 500 ml of ethyl ether and dried at 35°C in vacuo overnight to give 44.2 g (about 98% yield) of pure title compound.

3 - Preparation of $N^{63}$-carboxyamides of $N^{15}$-CBZ-teicoplanin $A_2$ complex.

A solution of 16 g (about 8 mmol) of $N^{15}$-CBZ-teicoplanin $A_2$ complex, cyanomethyl ester and of a large excess (from 50 to 100 mmol) of the proper reactant amine in 160 ml of DMF is stirred at room temperature for 60-120 min, afterwards 160 ml of absolute ethanol is added followed by 1.5 L of ethyl acetate. A solid separates which is collected by filtration and washed with 500 ml of ethyl ether, then it is dried at room temperature in the air to obtain a powder (yields generally > 85%) which is enough pure (HPLC titre generally > 90%) for the next hydrogenation step.

4 - Preparation of $N^{63}$-carboxyamides of teicoplanin $A_2$ complex.

The product obtained as described above (5 mmol) is dissolved in 500 ml of a mixture methanol : 0.04 N hydrochloric acid 7/3 (v/v) and the resulting solution is hydrogenated at room temperature and pressure in the presence of 5% Pd/C (5 g). As soon as the reaction is completed (HPLC) the catalyst is removed by filtration through a panel of celite (BDH 545). The clear filtrate is adjusted to pH 6.5 with 1 N NaOH and 500 ml of n-butanol is added. The resulting mixture is concentrated, at 40°C under reduced pressure, to a volume of about 150 ml, then 350 ml of ethyl ether are added and the precipitate is collected by filtration.

5 - Purification of the products by reverse phase column chromatography.

Crude products obtained as described above (10 g) are dissolved in a mixture (300 ml) acetonitrile : water 1:1 (v/v). Water is then added until a cloudy solution forms which is loaded at the top of a column of 500 g of silanized Silica-gel (0.06-0.2 mm; Merck Co.) prepared in the same solvent mixture (i.e., $CH_3CN$ and $H_2O$ in the ratio calculated on the base of the amount of $H_2O$ added to obtain the above cloudy solution) as obtained at the beginning of the precipitation. The column is developed with a linear gradient, from 10% to 80% of acetonitrile in water previously adjusted to pH 3.2 with glacial acetic acid, in 15 h at the rate of 400 ml/h while collecting 25 ml fractions which are monitored by HPLC. Those fractions containing the desired pure product are combined and enough n-butanol is added to obtain, after concentration at 45°C under vacuum a cloudy dry butanolic solution. On adding three volumes of ethyl ether a solid separates which is collected, washed with ethyl ether and dried at room temperature in vacuo overnight to give pure final compound.

The compounds of the invention (Table I) are thus obtained as the free bases (FB) when the only basic function present in the molecule is the free amino group in the position 15 of teicoplanin $A_2$ complex, or when the additional amino group introduced with the amide substituent is not enough basic to form an acid addition salt with acetic acid. Otherwise they are recovered as the acetates.

Preparation of the corresponding hydrochlorides is carried out, when this acid addition salt form is required, according to the following procedures:

1 mmol of an amide of teicoplanin $A_2$ complex, either as the free base or as the acetate, is dissolved in 10 ml of DMF. A 10% molar excess of 10 N HCl (0.11 ml for one amino function to be salified, 0.22 ml for two amino groups, etc.) is then added under stirring at 5°C, afterwards 40 ml of ethyl ether is added. The precipitate which forms is then collected by filtration, washed with ethyl ether and dried at room temperature in vacuo overnight (yields > 95%).

General Procedure B

1 - Preparation of $N^{15}$-tert-butyloxycarbonyl (t-BOC) deglucoteicoplanin.

To a stirred solution of 45 g (about 37 mmol) of antibiotic L 17392 (deglucoteicoplanin) in 600 ml of DMF, 19.3 g (about 65 mmol) of tert-butyl-2,4,5-trichlorophenylcarbonate and 10.2 ml (about 74 mmol) of TEA are added. The reaction mixture is stirred at room temperature for 24 h afterwards it is poured into 1.5 L of water. The resulting solution is adjusted to pH 3 with 1 N hydrochloric acid, then it is extracted with 3 L

of a mixture ethyl acetate : n-butanol 2:1 (v/v). The organic layer is separated, washed with 1 L of water, then it is concentrated at 40 °C under vacuum to a volume of about 300 ml. On adding 700 ml of ethyl ether, a solid separates which is collected by filtration, washed with 200 ml of ethyl ether and dried at room temperature in vacuo overnight, yielding 44 g (92%) of pure title compound.

2 - Preparation of $N^{15}$-t-BOC-deglucoteicoplanin, cyanomethyl ester.

A solution of 44 g (about 33 mmol) of $N^{15}$-t-BOC deglucoteicoplanin, 4.7 ml (about 34 mmol) of TEA and 44 ml of chloroacetonitrile in 440 ml of DMF is stirred at room temperature for 20 h, afterwards 1 L of ethyl acetate is added and the precipitate is collected by filtration. It is re-dissolved (about 46 g) in 1.5 L of a mixture methanol : water 1:2 (v/v) and the resulting solution is adjusted to pH 5.6 with glacial acetic acid. After adding 2 L of n-butanol, the most methanol is evaporated at 30 °C under vacuum and the organic layer is separated, washed with 1 L of water, then it is concentrated at 35 °C under vacuum to a final volume of about 300 ml. On adding 700 ml of ethyl ether, a solid separates which is collected by filtration, washed with 500 ml of ethyl ether, then it is dried at room temperature in vacuo overnight to give 42.5 g (96%) of pure title compound.

3 - Preparation of $N^{63}$-carboxyamides of $N^{15}$-t-BOC deglucoteicoplanin.

To a stirred solution of 14 g (about 10 mmol) of $N^{15}$-t-BOC-deglucoteicoplanin and of a large excess (from 100 to 150 mmol) of the proper reactant amine in 200 ml of DMF, 8.9 ml (about 150 mmol) of glacial acetic acid is added at room temperature. (The molar amount of glacial acetic acid depends on the structure of the reactant amine. In fact, for one mmol of amine, 0.5 mmol of glacial acetic acid is required when the amine does not contain additional basic functions, 1 mmol of glacial acetic acid when the amine contains one additional basic function, 1.5 mmol when the amine contain two additional basic functions, etc. Although the presence of acetic acid is unnecessary for the condensation, it is sometimes suitable to avoid side epimerization of the molecule at the $C^3$ position which might occur under basic conditions.

Furthermore, the presence of the acid does not influence the rate of the condensation reaction in the majority of cases).

After 3-6 h (the reaction, except a few cases, is in general completed within 3 h), 600 ml of ethyl acetate is added and the precipitate is collected by filtration, washed with 200 ml of ethyl ether and dried at room temperature in vacuo overnight to give a product which is enough pure for the next deprotection step (yields > 75%).

4 - Preparation of $N^{63}$-carboxyamides of deglucoteicoplanin.

A solution of 1 mmol of product obtained as described above, which has in general a HPLC titre > 85% and contains some acetate of the reactant amine as the main impurity, in 25-30 ml of anhydrous trifluoroacetic acid (TFA) is stirred at room temperature for 20 min, then solvent is evaporated at 25 °C under reduced pressure. The oily residue is re-dissolved in 50 ml of a mixture water : acetonitrile 6:4 (v/v) and the resulting solution is diluted with water until precipitation starts. The suspension thus obtained is adjusted to pH 3.O with 1 N hydrochloric acid (if necessary) and the resulting solution is loaded at the top of a column of 100 g of silanized Silica-gel (0.06-0.2 mm; Merck Co.) in water.

5 - Purification of the products by reverse-phase column chromatography.

The column loaded with the product, as described above, is developed with 1 L of water, then elution is performed with a linear gradient from 10% of acetonitrile in water to 50% of acetonitrile in 0.01 N hydrochloric acid, in 15 h at the rate of 200 ml/h, while collecting 10 ml fractions. Those fractions containing pure product are pooled and enough n-butanol is added to obtain, after concentration of the resulting mixture, a cloudy dry butanolic solution (30-100 ml). On adding three volumes of ethyl ether, a solid separates which is collected by filtration, washed with ethyl ether and dried at room temperature in vacuo for 2-3 days to yield pure final amides of deglucoteicoplanin as the hydrochlorides.

The corresponding trifluoroacetates are obtained by following the above chromatographic procedure of purification but eluting with a linear gradient from 10% to 60% of acetonitrile in water and maintaining the pH value of the eluent at 2.5 adding trifluoroacetic acid.

Example 1

a) Preparation of compound No. 1

A suspension of 2.5 g (about 1.25 mmol) of $N^{15}$-CBZ-teicoplanin $A_2$ complex, cyanomethyl ester in 125 ml of 13% ethanolic ammonia is stirred at room temperature for 3 h, afterwards solvents are evaporated at 40 °C under reduced pressure and the residue is slurried with 40 ml of ethyl ether to give 2.3 g of $N^{15}$-CBZ-compound 1. It is re-dissolved in 120 ml of a mixture methanol : water 8:2 (v/v) and the resulting solution is adjusted to pH 4.5 with 1 N HCl, then it is hydrogenated (1 atm, 20 °C) in the presence of 2 g of 5% Pd/C. After filtration, 50 ml of n-butanol is added and solvents are evaporated at 30 °C under vacuum. The crude residue (about 1.8 g) is re-dissolved in 30 ml of water : acetonitrile 85:15 (v/v) and chromatographed as described above in "general procedure A item 5" giving 1.25 g (49 % yield) of the title compound, as the hydrochloride.

b) Preparation of compound No.3

Treatment of 2.5 g (about 1.25 mmol) of $N^{15}$-CBZ-teicoplanin $A_2$ complex, cyanomethyl ester with 150 ml of 25% ethanolic methylamine under the above conditions (a) gives 2.15 g of $N^{15}$-CBZ-methylamide 3. Hydrogenation over 5% Pd/C (1.5 g) yields a crude product which is chromatographed to give 1.5 g (58% yield) of compound of the title, as the hydrochloride.

Example 2

a) Preparation of compound No. 2

A suspension of 2 g (about 1.5 mmol) of $N^{15}$-t-BOC-deglucoteicoplanin, cyanomethyl ester in 60 ml of 13% ethanolic ammonia is stirred at room temperature for 48 h, then solvents are evaporated at 45 °C under reduced pressure. The residue is slurried with 20 ml of ethyl ether and the crude $N^{15}$-t-BOC-compound 2 thus obtained (1.8 g) is re-dissolved in 10 ml of methylene chloride and treated with 6 ml of anhydrous trifluoroacetic acid (TFA) for 15 min. Solvents are evaporated at 35 °C under vacuum and the residue is chromatographed as described above in "general procedure B item 5" to give 0.87 g (41%) of the title compound, as the trifluoroacetate.

b) Preparation of compound No. 4

Treatment of 2 g (about 1.5 mmol) of $N^{15}$-t-BOC-deglucoteicoplanin, cyanomethyl ester with 150 ml of 25% ethanolic methylamine at room temperature for 2 h gives 1.9 g of crude $N^{15}$-t-BOC-compound 4. Deprotection (TFA) followed by chromatography of the resulting crude product are carried out as reported above (a) to yield 0.8 g (40%) of the title compound, as the trifluoroacetate.

Example 3

a) Preparation of compound No. 8

To a stirred solution of 6 g (about 3 mmol) of $N^{15}$-CBZ-teicoplanin $A_2$ complex, cyanomethyl ester in 60 ml of DMF, 3 ml of 1,3-propanediamine is added at room temperature. After 60 min, 60 ml of absolute ethanol is added, followed by 300 ml of ethyl acetate. A solid separates which is collected by filtration, washed with 150 ml of ethyl ether and dried at room temperature in vacuo overnight, yielding 6 g (100%) of pure $N^{15}$-CBZ of compound 8. It is dissolved in 450 ml of a mixture methanol : 0.04 N hydrochloric acid 7:3 (v/v) and the resulting solution is hydrogenated at room temperature and pressure in the presence of 3 g of 5% Pd/C. About 105 ml of $H_2$ are absorbed in 60 min, then the catalyst is filtered off and the clear filtrate is adjusted to pH 6.5 with 1 N NaOH.

The most methanol is evaporated at 30 °C under reduced pressure and the resulting cloudy aqueous solution is loaded at the top of a column of 600 g of silanized Silica-gel (0.06-0.2 mm, Merck Co.) in a mixture water : acetonitrile, 9:1 (v/v). The column is developed as described in "general procedure A item 5" and fractions containing pure components of compound 8 or mixtures thereof, are pooled and worked up to give 5.3 g (84%) of pure title compound, as the acetate.

b) Preparation of compound No. 18

To a stirred solution of 8 g (about 4 mmol) of $N^{15}$-CBZ-teicoplanin $A_2$ complex, cyanomethyl ester in 50 ml of DMF, a solution of 6 ml of N-ethylethylenediamine in 30 ml of DMF is added dropwise over a period of 30 min. After stirring at room temperature for 60 min, 120 ml of absolute ethanol is added and 6.5 g of pure $N^{15}$-CBZ-derivative of the title compound is obtained by precipitation from the resulting solution with 400 ml of ethyl acetate. Hydrogenation and purification by reverse-phase column chromatography as described above in Example 3a, yields 6 g (66%) of compound 18, as the acetate.

c) Preparation of compound No. 23

A solution of 15 g (about 7.5 mmol) of $N^{15}$-CBZ-teicoplanin $A_2$ complex, cyanomethyl ester and of 10 ml of 3-dimethylamino-1-propylamine in 150 ml of DMF is stirred it 15°-20°C for 45 min. After adding 100 ml of absolute ethanol followed by 1.5 L of ethyl acetate, a solid separates which is collected by filtration, washed with 400 ml of ethyl acetate, then with 200 ml of ethyl ether to give 14.9 g of $N^{15}$-CBZ-derivative of compound 23. It is re-dissolved in 500 ml of a mixture methanol : water 7:3 (v/v) and the resulting solution is adjusted to pH 3.5 with 1 N hydrochloric acid, afterwards it is hydrogenated at room temperature and pressure over 3.5 g of 5% Pd/C. After 90 min, (515 ml of $H_2$ is absorbed) the de-protection reaction at N-15 is completed (HPLC). The catalyst is filtered off and washed with 200 ml of a mixture methanol : water 1:1 (v/v). The filtrates are combined and the pH adjusted to 6.0 with 1 N NaOH, then the most methanol is evaporated at 35°C under reduced pressure. The cloudy solution thus obtained is adjusted to pH 8.3 with 1 N NaOH and the precipitated solid is collected by centrifugation, washed with 200 ml of water, then it is re-dissolved in 800 ml of a mixture water : methanol : n-butanol : glacial acetic acid 20:25:54:1. After concentration at 40°C under vacuum to a small volume (about 80 ml), ethyl ether (about 120 ml) is added and the precipitate is collected by filtration, washed with ethyl ether (about 400 ml), then it is dried in the air at room temperature for 3 days, yielding 13.0 g (85%) of pure compound 23 as the acetate. The same compound prepared according to the process of the European Patent Application Publ. No. 218099 showed a yield of about 46%.

d) Preparation of compound No. 32

1) The $N^{15}$-CBZ-teicoplanin $A_2$, component 2 is prepared from the component 2 of teicoplanin $A_2$ according to the same procedure described in "general procedure A, item 1", with the same yields.
2) The cyanomethyl ester of $N^{15}$-CBZ-teicoplanin $A_2$, component 2, is obtained from the component 2 of $N^{15}$-CBZ-teicoplanin $A_2$ according to the same procedure described in "general procedure A, item 2", substantially with the same yields.
3) A solution of 2 g (about 1 mmol) of the component 2 of $N^{15}$-CBZ-teicoplanin $A_2$, cyanomethyl ester and of 2 ml of 3-diethylamino-1-propylamine in 20 ml of DMF is stirred at room temperature for 20 min, afterwards 20 ml of absolute ethanol is added. On adding 80 ml of ethyl acetate, a solid separates which is collected and re-dissolved in 100 ml of a mixture methanol : 0.04 N hydrochloric acid 7:3 (v/v). The resulting solution is hydrogenated as described above in the "preparation of compound 23" to give 1.7 g (80%) of the compound of the title, as the hydrochloride. The same compound prepared according to the EPA Publ. No. 218099 showed a yield of about 54%.

e) Preparation of compound No. 43

A solution of 10 g (about 5 mmol) of $N^{15}$-CBZ-teicoplanin $A_2$ complex, cyanomethyl ester, and of 2.5 ml of N-methyl-piperazine in 100 ml of DMF is stirred at room temperature for 3 h, then 100 ml of absolute ethanol is added and stirring is continued for 2 h. On adding 300 ml of a mixture ethyl ether : ethyl acetate 3:2 (v/v), a solid separates which is collected by filtration, washed several time with 100 ml of ethyl acetate, and finally with 300 ml of ethyl ether. After drying in vacuo at room temperature for 3 days, 10.2 g of crude $N^{15}$-CBZ-derivative of compound 43 is obtained. Deprotection from CBZ group by catalytic hydrogenation followed by purification of the crude product thus obtained is carried out according to the "general procedure A/4,5" described above, yielding 8.5 g (82%) of the title compound, as the hydrochloride. The same compound prepared according to the process of the European Patent Publ. No. 218099 gives a yield of 47%.

f) Preparation of compound No. 47

To a stirred solution of 9.9 g (about 30 mmol) of $N_{epsilon}$CBZ-L-lysine methyl ester hydrochloride in 100 ml of DMF, 4.9 ml (about 35 mmol) of TEA followed by 10 g (about 5 mmol) of $N^{15}$-CBZ-teicoplanin $A_2$ complex, cyanomethyl ester is added at room temperature. After stirring at 15°-20°C overnight, the reaction mixture is poured into 900 ml of water. The resulting suspension is adjusted to pH 3.2 with 1 N hydrochloric acid and extracted with 1.5 L of n-butanol. The organic layer is separated, washed with 500 ml of water, then with 500 ml of a solution of 2% aqueous $NaHCO_3$ and finally with 1 L of water, afterwards it is concentrated to a small volume (about 100 ml). On adding ethyl ether (500 ml), a solid separates which is collected by filtration, washed with ethyl ether (300 ml), then it is dried in vacuo at room temperature overnight, yielding 8.9 g of crude $N^{15}$CBZ-derivative of $N_{epsilon}$-CBZ-compound 47. It is dissolved in 500 ml of a mixture methanol : 0.04 N hydrochloric acid 8:2 (v/v) and the resulting solution is hydrogenated at room temperature and pressure in the presence of 2.5 g of 5% Pd/C. After 60 min, 4 g of 5% Pd/C is added and hydrogenation is continued for 2 h (total amount of $H_2$ absorbed, about 630 ml). The catalyst is filtered off and washed with 200 ml of a mixture methanol : water 1:1 (v/v). The filtrates are combined and the resulting solution is adjusted to pH 6.3 with 1 N NaOH. After adding 1.2 L of n-butanol, the mixture is concentrated at 40°C under reduced pressure to a volume of about 100 ml. On adding 500 ml of ethyl acetate, a solid separates which is collected and purified by reverse-phase column chromatography as described in "general procedure A, item 5", thus obtaining 6.5 g (61%) of the title compound, as the acetate. The compound prepared according to the cited EPA Publ. No. 218099 showed a yield of about 38%.

Example 4

a) Preparation of compound No. 17

To a stirred solution of 5.6 g (about 4 mmol) of $N^{15}$-t-BOC-deglucoteicoplanin, cyanomethyl ester (prepared as described in "general procedure B/2") in 120 ml of DMF, 6.21 ml (about 60 mmol) of 3-methylamino-1-propylamine and 3.43 ml (about 60 mmol) of glacial acetic acid are added at room temperature. After 3 h, 120 ml of absolute ethanol is added followed by 260 ml of a mixture ethyl acetate : ethyl ether 1:1 (v/v). The precipitate is collected by centrifugation and suspended in 200 ml of absolute ethanol. After stirring at room temperature for 60 min, the insoluble matter is collected by filtration, washed with ethyl ether, then it is dried in vacuo at room temperature overnight, yielding 5.98 g of $N^{15}$-t-BOC-derivative of compound 17. It is re-dissolved in 60 ml of anhydrous trifluoroacetic acid and the resulting solution is stirred at room temperature for 20 min, afterwards the solvent is evaporated at 40°C under vacuum to give an oily residue which is chromatographed on a column of 400 g of silanized Silica-gel (0.06-0.2 mm, Merck Co.) as described in "general procedure B, item 5" to give 4.3 g (79%) of the title compound, as the dihydrochloride.

b) Preparation of compound No. 46

To a stirred solution of 5.6 g (about 4 mmol)of $N^{15}$-t-BOC-deglucoteicoplanin, cyanomethyl ester in 100 ml of DMF, 4.4 ml (about 50 mmol) of morpholine and 1.7 ml (about 30 mmol) of glacial acetic acid are added at room temperature. After 4 h, 100 ml of absolute ethanol is added and 5.7 g of crude $N^{15}$-t-BOC-derivative of compound 46 is obtained by precipitation with 400 ml of ethyl acetate. Deprotection at N-15 with TFA and purification carried out as above described in "example 4 a", yields 3.4 g (about 60% yield) of the title compound, as the trifluoroacetate (in this case, TFA is used in the chromatography instead of HCl). It is re-dissolved in 200 ml of a mixture water : n-butanol : methanol 4:4:2 (v/v/v) and the resulting solution is adjusted to pH 8.5 with 1 N NaOH. After adding 400 ml of a mixture water : n-butanol 1:1 (v/v), the organic layer is separated, washed twice with 300 ml of water (2 x 150 ml), afterwards it is concentrated at 45°C under vacuum to a small volume (about 50 ml). On adding ethyl ether (about 200 ml), a solid separates which is collected, washed with ethyl ether (200 ml) and dried in the air at room temperature overnight, to give 2.9 g (55%) of compound 46, as the free base. The yield of the same compound obtained according to the EPA Publ. No. 218099 was about 33%.

c) Preparation of compound No. 49

To a stirred solution of 9.9 g (about 30 mmol) of $N_{epsilon}$-CBZ-L-lysine methyl ester hydrochloride in 100 ml of DMF, 4.5 ml (about 30 mmol) of TEA and 8 g (about 6 mmol) of $N^{15}$-t-BOC-deglucoteicoplanin,

cyanomethyl ester are added at room temperature. Stirring is continued at 10°-15°C overnight, then 100 ml of absolute ethanol and 400 ml of ethyl ether are added. The crude solid which separates is collected by filtration and re-dissolved in 200 ml of anhydrous trifluoroacetic acid. After stirring at 25°C for 60 min, solvent is evaporated at 30°C under reduced pressure and the oily residue is re-dissolved in 400 ml of a mixture methanol : water 7:3 (v/v). The resulting solution is hydrogenated at room temperature and pressure over 5 g of 5% Pd/C for 90 min, the catalyst is filtered off and washed with 100 ml of a mixture methanol : water 1:1 (v/v). The filtrates are combined, 600 ml of n-butanol is added and the resulting solution is concentrated at 45°C under vacuum to a final volume of about 100 ml.

On adding 400 ml of ethyl acetate, a solid separates which is collected by filtration and re-dissolved in 200 ml of a mixture methanol : water 1:1 (v/v). After adding 50 g of silanized Silica-gel (0.06-0.2 mm; Merck Co.), the solvent of the suspension is evaporated under vacuum at 40°C. The residue is loaded at the top of a column of 800 g of silanized Silica-gel (0.06-0.2 mm; Merck Co.) in water. The column is then developed with a linear gradient from 10% of acetonitrile to 30% of acetonitrile in 0.001 N hydrochloric acid in 10 h at the rate of 400 ml/h, while collecting 15 ml fractions. Those fractions containing (HPLC) pure compound 49 are pooled and enough n-butanol is added to obtain, after concentration at 40°C under vacuum, a dry butanolic solution (about 100 ml) to which 0.5 ml of 37% hydrochloric acid is added followed by 400 ml of ethyl ether. The solid which separates is collected by filtration, washed with 200 ml of ethyl ether, then it is dried in vacuo at room temperature overnight, yielding 5.6 g (66%) of pure title compound, as the di-hydrochloride.

Example 5

Preparation of compounds 55-58

The $N^{15}$-CBZ derivative of compound 55 was prepared according to "General procedure A". A solution of crude $N^{15}$-CBZ-derivative of compound 55 (10.7 g, 3.07 mmol) in 350 ml of a mixture $CH_3OH$:0.001 N HCl 7:3 (v/v) was adjusted at pH 3 with 1 N HCl and hydrogenated (1 atm, room temperature) over 5 g of 5% Pd/C. About 270 ml of $H_2$ was absorbed within 70 min, the catalyst was filtered off through a panel of Celite BDH 545 filter aid and washed with 100 ml of a mixture $CH_3OH$:$H_2O$ 1:1 (v/v). The clear filtrates were combined and methanol was evaporated thus obtaining a aqueous solution (45 ml, pH 5.5) which was poured into 900 ml of acetone under vigorous stirring. The precipitated solid was collected by filtration, washed with 100 ml of acetone and dried in vacuo at room temperature overnight to yield 8.23 g (96%) of the title compound as the mono-hydrochloride (compound 56).

A solution of 2 g of this product in 8 ml of $H_2O$ was adjusted at pH 8.5 with 1 N NaOH and the precipitate was collected by filtration. It was washed with 2 ml of $H_2O$ and dried in vacuo at 30°C for 48 h to give 1.5 g (86%) of compound 55 as the free base.

A solution of 15 g of compound 56, obtained as described above, in 150 ml of water was adjusted at pH 6.5 with 1 N NaOH and loaded at the top of a column of 750 g of silanized Silica-gel (0.06-0.2 mm, Merck Co.) in water. The column was developed with 500 ml of $H_2O$, then with a linear gradient from 10% to 80% of $CH_3CN$ in $H_2O$, at pH 3.4 by $CH_3COOH$, in 20 h at a flow rate of 250 ml/h, while collecting 20 ml fractions which were checked by HPLC. Those fractions (86-110, 500 ml) containing the amide derivative with titre > 80% (which was expressed as the percentage of the areas of peaks) were pooled and 1 L of n-butanol was added. The resulting mixture was then concentrated to a final volume of about 150 ml and 350 ml of ethyl ether was added. The precipitate was collected, washed with ethyl ether and dried in the air at room temperature overnight to obtain 11 g (89%) of compound 58 as the mono-acetate.

To a stirred solution of 1 g of this product in 5 ml of water, 1.5 ml of 1 N HCl was added at 10°C, afterwards the resulting mixture was poured into 100 ml of acetone. The precipitate was collected and dried in vacuo at room temperature overnight to obtain 0.95 g (95%) of the di-hydrochloride (compound 57). An analytical pure sample of compound 55 is obtained according to the following procedure.

A solution of 9 g of compound 58 in 35 ml of water was brought to pH 8.7 with 1 N NaOH. A solid separated which was collected, washed with 5 ml of water and dried in the air at room temperature for 4 days to yield 7 g (78%) of compound 55 as the free base.

Example 6

Preparation of compound No. 62

a) Preparation of $N^{15}$-(1-methyl-2-hydroxy)ethyl teicoplanin.

To a stirred suspension of teicoplanin (6 g, 3 mmol) in methanol (180 ml), $NaBH_4$ pellet (6 g, 157 mmol) is added maintaining the reaction temperature at about 20 °C. The mixture is stirred at room temperature for 2 hours then 3-hydroxy-2-propanone (5 g, 55 mmol) is added. The reaction is continued overnight at the same temperature, then a second portion of 3-hydroxy-2-propanone (5 g, 55 mmol), followed after 1 hour by $NaBH_4$ (6 g) is added and the reaction continued for two hours. The mixture is diluted with methanol (120 ml), and $NaBH_4$ pellets (5 g) is added maintaining the temperature at below 30 °C. The mixture, adjusted to pH 5 with acetic acid, is concentrated under vacuum and the oily residue which forms is dissolved in 400 ml of water and the resulting solution is loaded at the top of a column of 600 g of silanized silica-gel (0.06-0.2 mm, Merck Co.). The column is eluted first with 3 1 of water and then with $CH_3CN/H_2O/CH_3COOH$ (45/45/10). The collected fractions are analyzed by HPLC.

The fractions containing > 90% (HPLC titre) of the $N^{15}$-alkyl derivative are combined, diluted with n-butanol and evaporated under vacuum at 50 °C. The residue is treated with ether, collected by filtration and dried, yielding 4.1 g of $N^{15}$-(1-methyl-2-hydroxy)ethyl teicoplanin.

b) Preparation of $N^{15}$-benzyl-$N^{15}$(1-methyl-2-hydroxy)ethyl teicoplanin derivative, 63-cyanomethyl ester.

A suspension of 2 mmol of $N^{15}$-(1-methyl--2-hydroxy)ethyl teicoplanin antibiotic, 0.3 ml (about 2.5 mmol) of benzyl bromide and 0.2 g (2 mmol) of potassium hydrogen carbonate in 75 ml of DMF is stirred at room temperature for 48 h, afterwards 0.4 ml (about 2.9 mmol) of TEA and 6 ml of chloroacetonitrile are added. The reaction mixture is stirred at room temperature overnight, then it is poured into 300 ml of ethyl acetate. The solid which separates is collected by filtration, washed with 100 ml of water, then with 300 ml of ethyl ether and dried in vacuo at room temperature overnight to give 1.8 mmol of crude $N^{15}$-benzyl-$N^{15}$-(1-methyl-2-hydroxy)ethyl teicoplanin derivative, 63-cyanomethyl ester.

c) Preparation of the title compound.

The active ester obtained above is dissolved in 50 ml of DMF, afterwards 20-25 mmol of $NH_2(CH_2)_3NH_2$ is added. The resulting solution is stirred at room temperature for 60 min, then 50 ml of ethanol is added. The mixture is diluted with 200 ml of water and the resulting solution is adjusted at pH 3.8 with 1 N HCl. It is then hydrogenated at room temperature and 1 atm over 2 g of 5% Pd/C.

As soon as about 120 ml of $H_2$ is absorbed, the catalyst is filtered off, the clear filtrate is adjusted at pH 7.9 with 1N NaOH, and the resulting solution is extracted with 500 ml of n-butanol. The organic layer is separated, washed with 200 ml of water and concentrated at 40 °C under reduced pressure to a final volume of about 70 ml. On adding 300 ml of ethyl acetate, a solid separates which is collected by filtration and dried in the air at room temperature for 3 days, to yield the title compound.

Example 7

preparation of compounds 59,63

a) By substantially following the procedure of Example 6-a but using teicoplanin aglycon instead of teicoplanin as the starting material, $N^{15}$-(1-methyl-2-hydroxy)ethyl deglucoteicoplanin derivative is obtained.

b) By substantially following the procedure of Example 6 b and c but using the proper amine reactant as reported in Table II the title compounds are obtained.

Example 8

Preparation of compound No. 60

a) Preparation of $N^{15}$-(2-dimethylamino)ethyl teicoplanin.

To a suspension of teicoplanin (2 g, 1.6 mmol) in methanol (60 ml), $NaBH_4$ (3.8 g, 100 mmol) is added

under a nitrogen atmosphere, while keeping the reaction temperature at about 40 ° C. The mixture is stirred 20 minutes then dimethylaminoacetaldehyde hydrochloride (1.5 g, 13.5 mmol) dissolved in methanol (20 ml) is added (dimethylaminoacetaldehyde hydrochloride is obtained from dimethylaminoacetaldehyde diethyl-acetal (Aldrich) by acidic hydrolysis according to J.H. Biel; W.K. Hoya and H.A. Leiser; J.A.C.S. 81, 2527; 1959). The reaction mixture is stirred two hours at room temperature, then cooled to 10 ° C, and a second portion of $NaBH_4$, (1.2 g, 31.7 mmol) is added thereto. The HPLC analysis after one hour shows that 30% of the starting antibiotic is still present.

Dimethylamino acetaldehyde hydrochloride (0.5 g, 4.5 mmol) and $NaBH_4$ (600 mg, 15.7 mmol) are then added. After standing overnight the reaction mixture is worked up by cooling the reaction mixture to 0 ° C, adjusting to pH 6 with 10% (w/w) HCl, and concentrated under reduced pressure. The crude material is suspended in water and loaded on a chromatographic column containing 100 g of silanized Silica-gel that is sequentially eluted with water (500 ml), 15% (w/w aqueous) $CH_3CN$ (500 ml) and a gradient $CH_3CN/H_2O$ from 15% to 25% acetonitrile in water. The fractions containing the compound of the title (HPLC content > 90%) are combined and concentrated under vacuum and the residue is treated with ethyl ether, collected by filtration and dried to give 1.1 g of the title compound.

b) By substantially following the same procedure of Example 6 b and c but starting from $N^{15}$-(2-dimethylamino)ethyl teicoplanin, instead of $N^{15}$-(1-methyl-2-hydroxy)ethyl teicoplanin, and from the proper amine, compound No. 60 is obtained.

Example 9

Preparation of compound No. 64

By substantially following the procedure of Example 6c) but reacting the $N^{15}$-benzyl-$N^{15}$-(1-methyl-2-hydroxy ethyl) teicoplanin derivative, 63 cyanomethyl ester of Example 6b) with $NH_2(CH_2)_3$-$N(CH_3)_2$ compound 64 is obtained.

Example 10

Preparation of compound No. 65

a) By substantially following the procedure of Example 6a) but using 3-hydroxy -2-butanone instead of 3-hydroxy-2-propanone as the starting material, $N^{15}$-(1-methyl-2-hydroxy-2 -methyl)ethyl) teicoplanin derivative is obtained.

b) By substantially following the procedure of Example 6b) and c) but using $NH_2(CH_2)_3N(CH_3)_2$ as amine reactant, compound No. 65 is obtained.

Example 11

Preparation of compound No. 66

a) By substantially following the procedure of Example 7a) but using teicoplanin aglycon instead of teicoplanin as the starting material, $N^{15}$-(1-methyl-2-hydroxy-2-methyl)ethyl deglucoteicoplanin derivative is obtained.

b) By following the procedure of Example 6b) and 6c) but using $NH_2(CH_2)_3$ $N(CH_3)_2$ as amine reactant, compound 66 is obtained.

## Claims

1. A process for preparing a compound of formula I

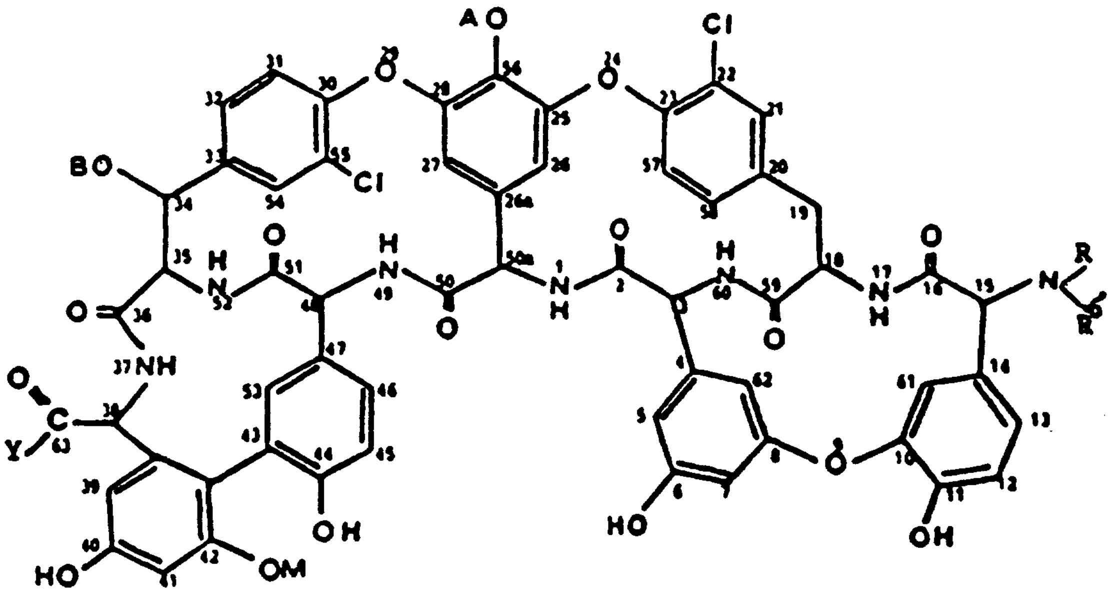

I

wherein:

R represents hydrogen; ($C_1$-$C_{12}$)alkyl; ($C_4$-$C_7$)cycloalkyl, cyano ($C_1$-$C_3$)alkyl; -$(CH_2)_q$-OOC-($C_1$-$C_6$)-alkyl; wherein q is an integer selected from 1, 2, 3 and 4; phenyl ($C_1$-$C_4$)alkyl wherein the phenyl group is optionally substituted in the position ortho, meta and/or para with 1 to 3 groups selected from ($C_1$-$C_4$)alkyl, nitro, bromo, chloro, iodo, ($C_1$-$C_4$)alkoxy, and phenyl;

$R^6$ represents hydrogen; ($C_1$-$C_{12}$)alkyl; ($C_4$-$C_7$)cycloalkyl; cyano ($C_1$-$C_3$)alkyl; -$(CH_2)_r$ -OOC-($C_1$-$C_6$)alkyl; wherein r is an integer selected from 1, 2, 3 and 4; phenyl ($C_1$-$C_4$)alkyl wherein the phenyl group is optionally substituted in the position ortho, meta and/or para with 1 to 3 groups selected from ($C_1$-$C_4$)alkyl, nitro, bromo, chloro, iodo, ($C_1$-$C_4$)alkoxy, and phenyl; or $R^6$ represents a group $[CHR^7 -(CR^8 R^9)_mX]_n-R^{10}$

wherein:

$R^7$ and $R^8$ independently represent H or a ($C_1$-$C_6$)alkyl;

$R^9$ represents H, a ($C_1$-$C_6$) alkyl or OH;

$R^{10}$ represents H, a ($C_1$-$C_3$)alkyl, $COOR^{11}$, $OR^{11}$, $SR^{11}$, $NR^{11}R^{12}$ or halogen;

$R^{11}$ and $R^{12}$ independently represent H or a ($C_1$-$C_3$)alkyl; m is zero or 1, n is an integer comprised between zero and 6, extremes included.

X is O, NH or a bond with the proviso that when X is O or NH, then n is different from zero, and $R^9$ is different from OH with the proviso that when one between R and $R^6$ represents $(CH_2)_n$-OOC-($C_1$-$C_6$)-alkyl the other must represent hydrogen;

Y represents a group

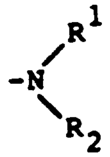

wherein

$R^1$ represents hydrogen, ($C_1$-$C_6$)alkyl, hydroxy-($C_2$-$C_4$)alkyl, halogen ($C_2$-$C_4$)alkyl, ($C_1$-$C_4$)alkoxy, ($C_2$-$C_4$)alkyl, amino ($C_2$-$C_4$)alkyl, ($C_1$-$C_4$)alkylamino ($C_2$-$C_4$)alkyl, di($C_1$-$C_4$)alkylamino ($C_2$-$C_4$)alkyl;

$R^2$ represents hydrogen, ($C_1$-$C_6$)alkyl, hydroxy-($C_2$-$C_4$)alkyl, halogen ($C_2$-$C_4$)alkyl, ($C_1$-$C_4$)alkoxy

($C_2$-$C_4$)alkyl, a nitrogen containing 5-6 membered heterocyclic ring which may be unsaturated, partially saturated or wholly saturated and may contain 1 to 3 further heteroatoms selected from N, S and O wherein 1 to 3 of the ring carbons may optionally bear ($C_1$-$C_4$)alkyl substituents and one of the nitrogen rings may optionally bear a substituent $R^5$ selected from ($C_1$-$C_4$)alkyl, ($C_4$-$C_7$)cycloalkyl, phenyl optionally substituted with halogen or ($C_1$-$C_4$)alkyl, phenyl ($C_1$-$C_4$)alkyl, pyridyl, ($C_1$-$C_4$)alkylpyridinio, and when the ring is wholly saturated two of the ring members may optionally be bridged by an alkylene chain of 1 to 3 carbon atoms wherein one of the methylene groups may optionally be replaced by -NH- or -N[($C_1$-$C_4$)alkyl]; a group -alk-W wherein "alk" represents a linear alkylene chain of 1 to 8 carbon atoms which is optionally substituted with a substituent selected from ($C_1$-$C_4$)alkyl, hydroxy-($C_1$-$C_4$)alkyl, hydroxy, carboxyaminocarbonyl, ($C_1$-$C_4$)alkylaminocarbonyl, di($C_1$-$C_4$)alkylaminocarbonyl, ($C_1$-$C_4$)alkylcarbonyl, phenyl ($C_1$-$C_4$)alkoxycarbonyl, and W represents a carboxy, ($C_1$-$C_4$)alkoxycarbonyl, phenyl ($C_1$-$C_4$)alkoxycarbonyl, aminocarbonyl, ($C_1$-$C_4$)alkylaminocarbonyl, di($C_1$-$C_4$)alkylaminocarbonyl, pentosaminocarbonyl, hexosaminocarbonyl, ureido, guanidino, a nitrogen containing 5-6 membered heterocyclic ring defined as above, a group of the formula

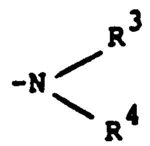

wherein $R^3$ and $R^4$ each independently represents hydrogen, ($C_1$-$C_6$)alkyl, hydroxy ($C_2$-$C_4$)alkyl and halogen ($C_2$-$C_4$)alkyl, or $R^4$ represents phenylmethyloxycarbonyl and $R^3$ represents hydrogen; or $R^1$ and $R^2$ taken together with the adjacent nitrogen atom represent a saturated 5-7 membered heterocyclic ring which may optionally bear one to two ($C_1$-$C_4$)alkyl substituents on the ring carbons and may contain a further heterogroup selected from -O-,-S-, and -$NR^5$-wherein $R^5$ is defined as above;

A represents hydrogen or -N[$C_9$-$C_{12}$)aliphatic acyl]-beta-D-2-deoxy-2-amino-glucopyranosyl;

B represents hydrogen or N-acetyl-beta-D-2-deoxy-2-aminoglucopyranosyl;

M represents hydrogen or alpha-D-mannopyranosyl; with the proviso that B represents hydrogen only when A and M are simultaneously hydrogen, and with the further proviso that when W represents a group

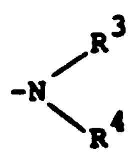 ,

ureido, guanidino or a nitrogen containing 5-6 membered heterocyclic ring as defined above directly connected with the "alk" moiety through a bond with a ring nitrogen atom, the linear alkylene "alk" moiety must be of at least two carbon atoms; and the pharmaceutically acceptable addition salts thereof, which comprises reacting a compound of formula II

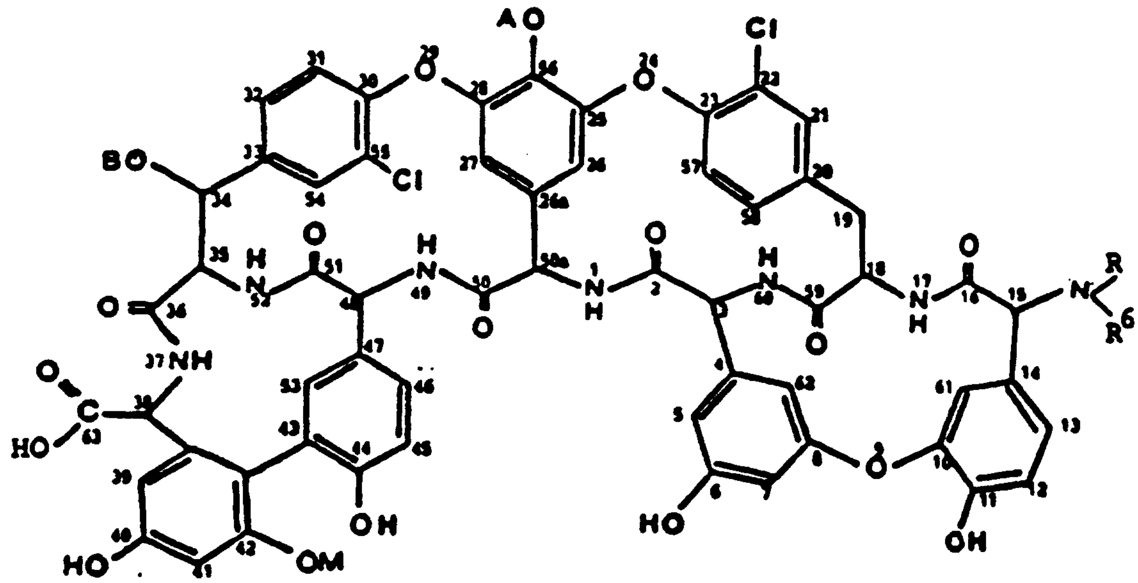

II

wherein:

A, B, M, R and $R^6$ are as defined, protected on the 15 amino function by a $N^{15}$-protecting group when at least one of R and $R^6$ is H, with an activated ester forming reagent to give an activated ester function which is substituted by an amine of formula $NHR^1R^2$ wherein $R^1$ and $R^2$ are as defined, by contacting with an excess of said amine in an organic polar solvent at a temperature comprised between 5 ° C and 60 ° C.

**2.** A process as in claim 1 wherein the $N^{15}$-protecting group is selected from carbamate forming reagents characterized by the following oxycarbonyl groups: 1,1-dimethylpropynyloxycarbonyl, t-butyloxycarbonyl, vinyloxycarbonyl, aryloycarbonyl, cinnamyloxycarbonyl,benzyloxycarbonyl, p-nitrobenzyloxycarbonyl, 3,4-dimethoxy-6-nitrobenzyloxycarbonyl, 2,4-dichlorobenzyloxycarbonyl, 5-benzisoxazolylmethyloxycarbonyl, 9-anthranylmethyloxycarbonyl, diphenylmethyloxycarbonyl, isonicotinyloxycarbonyl, S-benzyloxycarbonyl, or aldehydes and ketones which are capable of forming Schiff bases with the $N^{15}$-amino group of the teicoplanin moiety.

**3.** A process as in claim 2 wherein the $N^{15}$-protecting groups are selected from benzyloxycarbonyl and t-butyloxy carbonyl.

**4.** A process as in claims 1, 2 or 3 wherein the activated ester forming reagents are selected from $ClCH_2CN$, $BrCH_2COOC_2H_5$, $BrCH(COOC_2H_5)_2$, $ClCH_2COCH_3$,

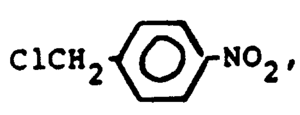, $ClCH_2CH_2N(C_2H_5)_2$.

**5.** A process as in any of claims 1 to 4 wherein the activated ester forming reagent is $ClCH_2CN$.

**6.** A process as in any of claims 1 to 5 wherein the organic polar solvent is selected from $C_1$-$C_4$ alkanols, N,N-dimethylformamide, hexamethyl phosphoramide, 1,3-dimethyl-3,4,5,6-tetrahydro-2(1H)-pyrimidinone (DMPU), dimethylsulfoxyde, dimethoxyethane or mixtures thereof.

**7.** A process as any of claims 1 to 6 further characterized in that the $N^{15}$-protecting group is removed at the end of the condensation reaction by catalytic hydrogenation or by selective displacement under acidic conditions which do not affect the other functions in the desired amide derivative compound.

**8.** A process as in claim 7 wherein the catalytic hydrogenation is carried out in the presence of palladium on carbon.

**9.** A process as in claim 7 wherein the selective displacement is carried out in the presence of anhydrous trifluoroacetic acid at room temperature.

**10.** A process as in any of claims 1 to 9 wherein the excess of the amine reactant is comprised between 1:5 and 1:40 with respect to the activated ester derivative.

**11.** A process as in claim 10 wherein the excess of the amine reactant is 1 to 10 with respect to the activated ester derivative.

**12.** A process according to anyone of claims 1 to 11 for preparing a compound of formula I wherein:

a) A is N[($C_9$-$C_{11}$)aliphatic acyl]-beta-D-2-deoxy-2-aminoglucopyranosyl;
B is N-acetyl-beta-D-2-deoxy-2-aminoglucopyranosyl;
M is alpha-D-mannopyranosyl;
R and $R^6$ are hydrogen atoms and
Y is $NH_2$, $NHCH_3$, $NH(CH_2)_2NH_2$, $NH(CH_2)_3NH_2$, $NH(CH_2)_2NHCH_3$, $NH(CH_2)_3NHCH_3$, $NH(CH_2)_2NH(C_2H_5)$, $NH(CH_2)_3NH(C_2H_5)$, $N(CH_3)(CH_2)_2NHCH_3$, $N(CH_3)(CH_2)_3NHCH_3$, $NHCH(COOCH_3)(CH_2)_4NH_2$, $NH(CH_2)_3NH(CH_2)_2OH$ or $NH(CH_2)_3N(CH_3)_2$;

b) A is N-[($C_9$-$C_{11}$)aliphatic acyl]-beta-D-2-deoxy-2-aminoglucopyranosyl;
B is N-acetyl-beta-D-2-deoxy-2-aminoglucopyranosyl;
M is alpha-D-mannopyranosyl
R is hydrogen, $R^6$ is $CH_2CH_2$-$N(CH_3)_2$ and
Y is $NH(CH_2)_3N(CH_3)_2$;

c) A is N-(8-methylnonanoyl)-beta-D-2-deoxy-2-aminoglucopyranosyl;
B is N-acetyl beta-D-2-deoxy-2-aminoglucopyranosyl;
M is alpha D-mannopyranosyl;
R and $R^6$ are hydrogen atoms and
Y is $NH(CH_2)_3$-$N(CH_3)_2$;

d) A, B, M, R and $R^6$ are hydrogen atoms and
Y is $NH_2$, $NHCH_3$, $NH(CH_2)_2NH_2$, $NH(CH_2)_3NH_2$, $NH(CH_2)_4NH_2$, $NH(CH_2)_6NH_2$, $NH(CH_2)_2NHCH_3$, $NH(CH_2)_3NHCH_3$, $NH(CH_2)_2NHC_2H_5$, $NH(CH_2)_5N(CH_3)_2$, $NH(CH_2)_7N(CH_3)_2$, $N(CH_3)(CH_2)_2NHCH_3$, $N(CH_3)(CH_2)_3NHCH_3$ and $NH(CH_2)_3NH(CH_2)_2OH$;

e) A, B, M and R are hydrogen atoms, $R^6$ is $CH(CH_3)CH(CH_3)OH$ and Y is $NH(CH_2)_3$-$N(CH_3)_2$;

and the pharmaceutically acceptable salts thereof.

**13.** A Process according to anyone of claims 1 to 11 for preparing the compound of formula I that is $C^{63}$3-dimethylamino-1-propylamide teicoplanin $A_2$ component 2 and its pharmaceutically acceptable salts.

**Patentansprüche**

**1.** Verfahren zur Herstellung einer Verbindung der Formel I

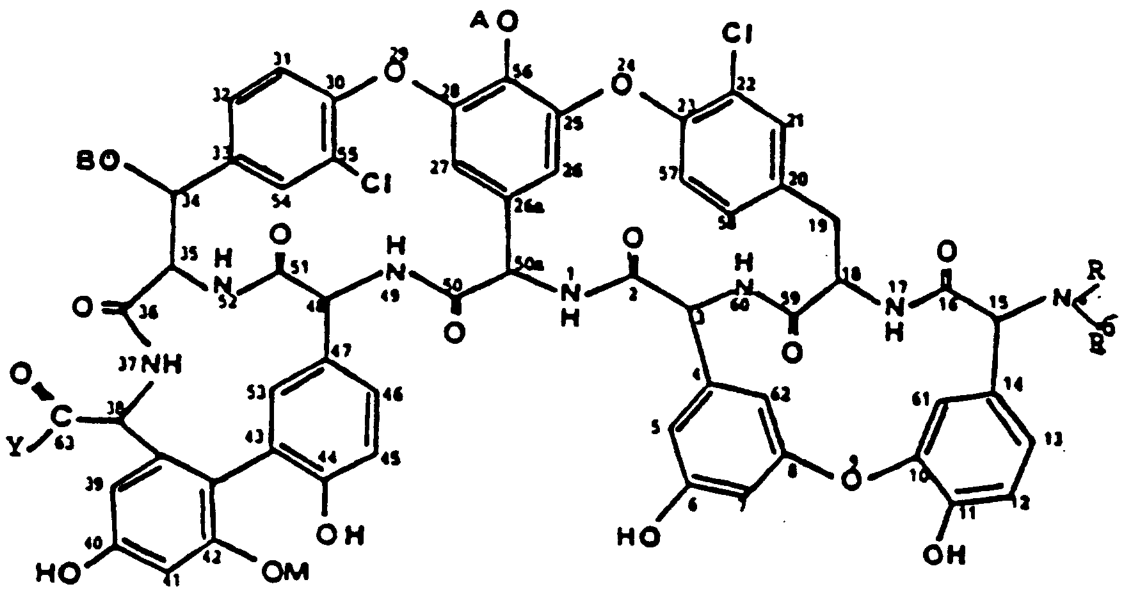

I

in der

R ein Wasserstoffatom, einen $(C_1\text{-}C_{12})$Alkyl-, $(C_4\text{-}C_7)$Cycloalkyl-, Cyano$(C_1\text{-}C_3)$alkyl- oder $-(CH_2)_q$-OOC-$(C_1\text{-}C_6)$alkylrest, in dem q eine ganze Zahl, ausgewählt aus 1, 2, 3 oder 4 bedeutet, einen Phenyl-$(C_1\text{-}C_4)$alkylrest, in dem der Phenylrest gegebenenfalls in ortho-, meta- und/oder para-Stellung mit 1 bis 3 Resten, ausgewählt aus $(C_1\text{-}C_4)$Alkylresten, Nitrogruppen, Brom-, Chlor- oder Iodatomen, $(C_1\text{-}C_4)$-Alkoxyresten oder Phenylgruppen, substituiert ist, bedeutet;

$R^6$ ein Wasserstoffatom, einen $(C_1\text{-}C_{12})$Alkyl-, $(C_4\text{-}C_7)$Cycloalkyl-, Cyano$(C_1\text{-}C_3)$alkyl- oder $-(CH_2)_r$-OOC-$(C_1\text{-}C_6)$alkylrest, in dem r eine ganze Zahl, ausgewählt aus 1, 2, 3 oder 4 bedeutet, einen Phenyl-$(C_1\text{-}C_4)$alkylrest, in dem der Phenylrest gegebenenfalls in ortho-, meta- und/oder para-Stellung mit 1 bis 3 Resten, ausgewählt aus $(C_1\text{-}C_4)$Alkylresten, Nitrogruppen, Brom-, Chlor- oder Iodatomen, $(C_1\text{-}C_4)$-Alkoxyresten oder Phenylgruppen, substituiert ist, bedeutet; oder $R^6$ einen Rest $[CHR^7(CR^8R^9)_mX]_n$-$R^{10}$ bedeutet, in dem

$R^7$ und $R^8$ unabhängig voneinander ein Wasserstoffatom oder einen $(C_1\text{-}C_6)$Alkylrest bedeuten;

$R^9$ ein Wasserstoffatom, einen $(C_1\text{-}C_6)$Alkylrest oder eine OH-Gruppe bedeutet;

$R^{10}$ ein Wasserstoffatom, einen $(C_1\text{-}C_3)$Alkylrest, einen Rest $COOR^{11}$, $OR^{11}$, $SR^{11}$, $NR^{11}R^{12}$ oder ein Halogenatom bedeutet;

$R^{11}$ und $R^{12}$ unabhängig voneinander ein Wasserstoffatom oder einen $(C_1\text{-}C_3)$Alkylrest bedeuten; m den Wert 0 oder 1 hat und n eine ganze Zahl zwischen 0 und 6 bedeutet, Randwerte eingeschlossen;

X ein O-Atom, eine NH-Gruppe oder eine Bindung bedeutet, mit der Maßgabe, daß, wenn X ein O-Atom oder eine NH-Gruppe bedeutet, n von 0 verschieden ist und $R^9$ von einer OH-Gruppe verschieden ist; mit der Maßgabe, daß, wenn einer der Reste R oder $R^6$ einen $(CH_2)_n$-OOC-$(C_1\text{-}C_6)$Alkylrest bedeutet, der andere Rest ein Wasserstoffatom darstellen muß;

Y einen Rest

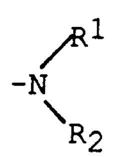

bedeutet, in dem $R^1$ ein Wasserstoffatom, einen $(C_1\text{-}C_6)$Alkyl-, Hydroxy-$(C_2\text{-}C_4)$alkyl-, Halogen$(C_2\text{-}C_4)$-alkyl-, $(C_1\text{-}C_4)$Alkoxy-, $(C_2\text{-}C_4)$Alkyl-, Amino$(C_2\text{-}C_4)$alkyl-, $(C_1\text{-}C_4)$Alkylamino$(C_2\text{-}C_4)$alkyl- oder di$(C_1$-

$C_4$)Alkylamino($C_2$-$C_4$)alkylrest bedeutet;

$R^2$ ein Wasserstoffatom, einen ($C_1$-$C_6$)Alkyl-, Hydroxy-($C_2$-$C_4$)alkyl-, Halogen($C_2$-$C_4$)alkyl, ($C_1$-$C_4$)-Alkoxy($C_2$-$C_4$)alkylrest, einen stickstoffhaltigen 5-6-gliedrigen heterocyclischen Ring, der ungesättigt, teilweise gesättigt oder vollständig gesättigt sein und 1 bis 3 weitere Heteroatome, ausgewählt aus einem N-, S- oder O-Atom, enthalten kann, wobei 1 bis 3 Ringkohlenstoffatome gegebenenfalls ($C_1$-$C_4$)-Alkylsubstituenten tragen können und eines der Ringstickstoffatome gegebenenfalls einen Substituenten $R^5$ tragen kann, der ausgewählt ist aus einem ($C_1$-$C_4$)Alkyl-, ($C_4$-$C_7$)Cycloalkylrest oder eine Phenylgruppe, gegebenenfalls mit einem Halogenatom oder einem ($C_1$-$C_4$)Alkyl-, Phenyl($C_1$-$C_4$)alkyl-, Pyridyl- oder ($C_1$-$C_4$)Alkylpyridinorest substituiert, wobei, wenn der Ring ganz gesättigt ist, zwei der Ringatome gegebenenfalls durch eine Alkylenkette mit 1 bis 3 Kohlenstoffatomen verbrückt sein können, wobei eine der Methylengruppen gegebenenfalls ersetzt werden kann durch einen Rest -NH- oder -N[($C_1$-$C_4$)Alkyl]; einen Rest -Alk-W bedeutet, in dem "Alk" eine lineare Alkylenkette mit 1 bis 8 Kohlenstoffatomen bedeutet, die gegebenenfalls mit einem Substituenten, ausgewählt aus einem ($C_1$-$C_4$)Alkyl-, Hydroxy($C_1$-$C_4$)alkyl-, Hydroxy-, Carboxyaminocarbonyl-, ($C_1$-$C_4$)Alkylaminocarbonyl-, di($C_1$-$C_4$)Alkylaminocarbonyl-, ($C_1$-$C_4$)Alkylcarbonyl- oder Phenyl($C_1$-$C_4$)alkoxycarbonylrest substituiert ist und W einen Carboxy-, ($C_1$-$C_4$)Alkoxycarbonyl-, Phenyl($C_1$-$C_4$)alkoxycarbonyl-, Aminocarbonyl-, ($C_1$-$C_4$)Alkylaminocarbonyl-, di($C_1$-$C_4$)Alkylaminocarbonyl-, Pentosaminocarbonyl-, Hexosaminocarbonyl-, Ureido- oder Guanidinorest, einen stickstoffhaltigen 5-6-gliedrigen heterocyclischen Ring mit der vorstehenden Bedeutung oder einen Rest der Formel

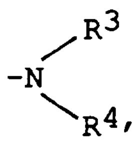

in dem $R^3$ und $R^4$ unabhängig voneinander jeweils ein Wasserstoffatom, einen ($C_1$-$C_6$)Alkyl-, Hydroxy-($C_2$-$C_4$)alkyl- oder Halogen($C_2$-$C_4$)alkylrest bedeuten oder $R^4$ eine Phenylmethyloxycarbonylgruppe und $R^3$ ein Wasserstoffatom bedeutet, darstellt; oder

$R^1$ und $R^2$ zusammen mit dem benachbarten Stickstoffatom einen gesättigten 5-7-gliedrigen heterocyclischen Ring bedeuten, der gegebenenfalls einen oder zwei ($C_1$-$C_4$)Alkylsubstituenten an den Ringkohlenstoffatomen trägt und einen weiteren Heterorest enthalten kann, der ausgewählt ist aus den Resten -O-, -S- und $NR^5$-, wobei $R^5$ die vorstehende Bedeutung hat;

A ein Wasserstoffatom oder einen -N[aliphatischen($C_9$-$C_{12}$)-Acyl]-$\beta$-D-2-desoxy-2-aminoglucopyranosylrest bedeutet;

B ein Wasserstoffatom oder einen -N-Acetyl-$\beta$-D-2-desoxy-2-amino-glucopyranosylrest bedeutet;

M ein Wasserstoffatom oder einen $\alpha$-D-Mannopyranosylrest bedeutet; mit der Maßgabe, daß B nur dann ein Wasserstoffatom bedeutet, wenn A und M gleichzeitig Wasserstoffatome bedeuten und mit der weiteren Maßgabe, daß, wenn W einen Rest

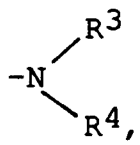

einen Ureido-, Guanidinorest oder einen stickstoffhaltigen 5-6-gliedrigen heterocyclischen Ring, mit der vorsthenden Bedeutung darstellt, der über eine Bindung mit einem Ringstickstoffatom direkt mit der "Alk"-Komponente verbunden ist, die lineare Alkylen-"Alk"-Komponente aus mindestens zwei Kohlenstoffatomen bestehen muß;
und der pharmazeutisch verträglichen Additionssalze davon; umfassend die Umsetzung einer Verbindung der Formel II

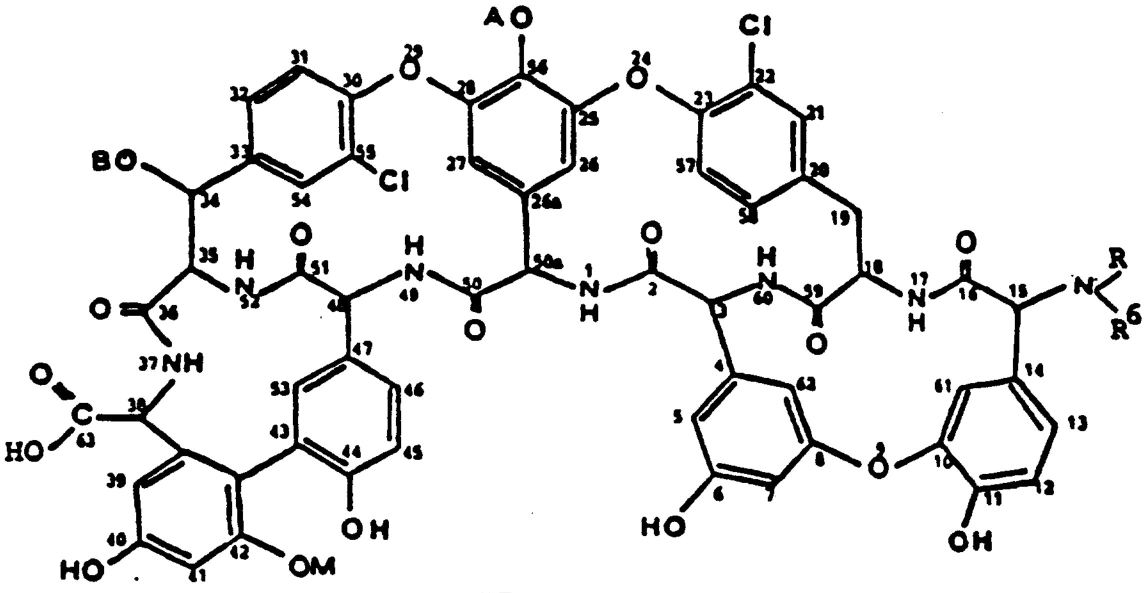

II

in der

A, B, M, R und $R^6$ die vorstehenden Bedeutungen haben und die, falls mindestens einer der Reste R und $R^6$ ein Wasserstoffatom ist, an der 15-Aminofunktion durch eine $N^{15}$-Schutzgruppe geschützt ist, mit einem, einen aktivierten Ester bildenden Reagenz, wobei eine aktivierte Esterfunktion erhalten wird, die durch ein Amin der Formel $NHR^1R^2$, in der die Reste $R^1$ und $R^2$ die vorstehende Bedeutung haben, substituiert wird, indem sie mit einem Überschuß des Amins in einem polaren organischen Lösungsmittel bei einer Temperatur zwischen 5 ° C und 60 ° C in Kontakt gebracht wird.

**2.** Verfahren nach Anspruch 1, wobei die $N^{15}$-Schutzgruppe ausgewählt ist aus Carbamat erzeugenden Reagentien, welche durch die folgenden Oxycarbonylgruppen gekennzeichnet sind:
1,1-Dimethylpropinyloxycarbonyl-, t-Butyloxycarbonyl-, Vinyloxycarbonyl-, Aryloxycarbonyl-, Cinnamyloxycarbonyl-, Benzyloxycarbonyl-, p-Nitrobenzyloxycarbonyl-, 3,4-Dimethoxy-6-nitrobenzyloxycarbonyl-, 2,4-Dichlorbenzyloxycarbonyl-, 5-Benzisoxazolylmethyloxycarbonyl-, 9-Anthranylmethyloxycarbonyl-, Diphenylmethyloxycarbonyl-, Isonicotinyloxycarbonyl-, S-Benzyloxycarbonylgruppen oder Aldehyde oder Ketone, die in der Lage sind, mit der $N^{15}$-Aminogruppe der Teicoplaninkomponente Schiff sche Basen zu bilden.

**3.** Verfahren nach Anspruch 2, wobei die $N^{15}$-Schutzgruppen ausgewählt sind aus einer Benzyloxycarbonyl- und t-Butyloxycarbonylgruppe.

**4.** Verfahren nach den Ansprüchen 1, 2, oder 3, wobei die einen aktivierten Ester bildenden Reagentien ausgewählt sind aus den Verbindungen $ClCH_2CN$, $BrCH_2COOC_2H_5$, $BrCH(COOC_2H_5)_2$, $ClCH_2COCH_3$,

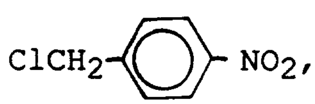, $ClCH_2CH_2N(C_2H_5)_2$.

**5.** Verfahren nach einem der Ansprüche 1 bis 4, wobei das einen aktivierten Ester bildende Reagens $ClCH_2CN$ ist.

**6.** Verfahren nach einem der Ansprüche 1 bis 5, wobei das polare organische Lösungsmittel ausgewählt ist aus $(C_1$-$C_4)$Alkanolen, N,N-Dimethylformamid, Hexamethylphosphoramid, 1,3-Dimethyl-3,4,5,6-tetrahydro-2(1H)-pyrimidinon (DMPU), Dimethylsulfoxid, Dimethoxyethan oder deren Gemische.

7. Verfahren nach einem der Ansprüche 1 bis 6, weiterhin dadurch gekennzeichnet, daß die $N^{15}$-Schutzgruppe am Ende der Kondensationsreaktion durch katalytische Hydrierung oder durch selektives Ersetzen unter sauren Bedingungen entfernt wird, wobei die anderen Funktionen im gewünschten Amidderivat nicht beeinträchtigt werden.

8. Verfahren nach Anspruch 7, wobei die katalytische Hydrierung in Gegenwart von Palladium auf Kohlenstoff durchgeführt wird.

9. Verfahren nach Anspruch 7, wobei das selektive Ersetzen in Gegenwart von wasserfreier Trifluoressigsäure bei Raumtemperatur durchgeführt wird.

10. Verfahren nach einem der Ansprüche 1 bis 9, wobei der Überschuß des Aminreaktanten zwischen 1:5 und 1:40 beträgt, bezogen auf das aktivierte Esterderivat.

11. Verfahren nach Anspruch 10, wobei der Überschuß des Aminreaktanten 1 zu 10 beträgt, bezogen auf das aktivierte Esterderivat.

12. Verfahren nach einem der Anprüche 1 bis 11 zur Herstellung einer Verbindung der Formel I, in der
a) A einen N[aliphatischen($C_9$-$C_{11}$)Acyl]-$\beta$-D-2-desoxy-2-aminoglucopyranosylrest bedeutet;
B einen N-Acetyl-$\beta$-D-2-desoxy-2-aminoglucopyranosylrest bedeutet;
M einen $\alpha$-D-Mannopyranosylrest bedeutet;
R und $R^6$ Wasserstoffatome bedeuten und
Y eine Gruppe $-NH_2$, $-NHCH_3$, $-NH(CH_2)_2NH_2$, $-NH(CH_2)_3NH_2$, $-NH(CH_2)_2NHCH_3$, $-NH(CH_2)_3NHCH_3$,$-NH(CH_2)_2NH(C_2H_5)$, $-NH(CH_2)_3NH(C_2H_5)$, $-N(CH_3)(CH_2)_2NHCH_3$, $-N(CH_3)(CH_2)_3NHCH_3$, $-NHCH(COOCH_3)(CH_2)_4NH_2$, $-NH(CH_2)_3NH(CH_2)_2OH$ oder $NH(CH_2)_3N(CH_3)_2$ bedeutet;
b) A einen N[aliphatischen($C_9$-$C_{11}$)Acyl]-$\beta$-D-2-desoxy-2 aminoglucopyranosylrest bedeutet;
B einen N-Acetyl-$\beta$-D-2-desoxy-2-aminoglucopyranosylrest bedeutet;
M einen $\alpha$-D-Mannopyranosylrest bedeutet;
R ein Wasserstoffatom, $R^6$ eine $-CH_2CH_2-N(CH_3)_2$ Gruppe und
Y eine $-NH(CH_2)_3N(CH_3)_2$ Gruppe bedeutet
c) A einen N-(8-Methylnonanoyl)-$\beta$-D-2-desoxy-2-aminoglucopyranosylrest bedeutet;
B einen N-Acetyl-$\beta$-D-2-desoxy-2-aminoglucopyranosylrest bedeutet;
M einen $\alpha$-D-Mannopyranosylrest bedeutet;
R und $R^6$ Wasserstoffatome bedeuten und
Y eine $-NH(CH_2)_3N(CH_3)_2$ Gruppe bedeutet
d) A, B, M, R und $R^6$ Wasserstoffatome bedeuten und
Y eine Gruppe $-NH_2$, $-NHCH_3$, $-NH(CH_2)_2NH_2$, $-NH(CH_2)_3NH_2$, $-NH(CH_2)_4NH_2$, $-NH(CH_2)_6NH_2$, $-NH(CH_2)_2NHCH_3$, $-NH(CH_2)_3NHCH_3$-, $-NH(CH_2)_2NHC_2H_5$, $-NH(CH_2)_5N(CH_3)_2$, $-NH(CH_2)_7N(CH_3)_2$, $-N(CH_3)(CH_2)_2NHCH_3$, $-N(CH_3)(CH_2)_3NHCH_3$ oder $-NH(CH_2)_3NH(CH_2)_2OH$ bedeutet;
e) A, B, M und R Wasserstoffatome bedeuten, $R^6$ eine $CH(CH_3)CH(CH_3)OH$-Gruppe und Y eine $-NH(CH_2)_3-N(CH_3)_2$ Gruppe bedeutet;
und deren pharmazeutisch verträgliche Salze.

13. Verfahren nach einem der Ansprüche 1 bis 11 zur Herstellung der Verbindung der Formel I, nämlich der Komponente 2 von $C^{63}$-3-Dimethylamino-1-propylamid-teicoplanin $A_2$, und deren pharmazeutisch verträglicher Salze.

## Revendications

**1.** Procédé de préparation d'un composé répondant à la formule I

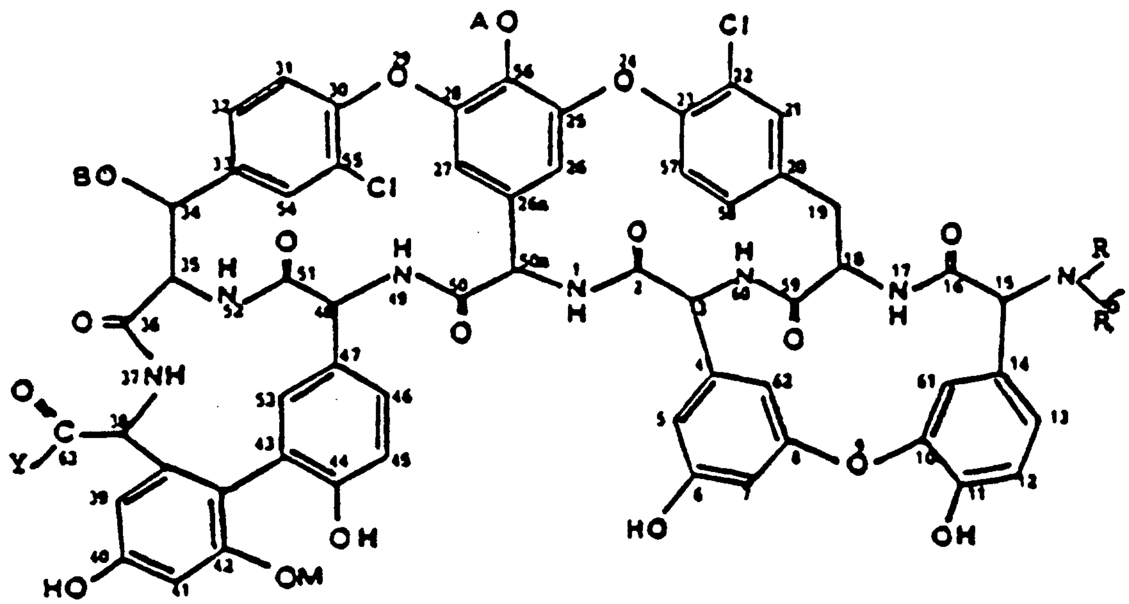

I

dans laquelle :

R représente un atome d'hydrogène ; un groupe alkyle en $C_1$-$C_{12}$ ; cycloalkyle en $C_4$-$C_7$, cyano-(alkyle en $C_1$-$C_3$) ; -$(CH_2)_q$-OOC-(alkyle en $C_1$-$C_6$) ; où q est un entier choisi parmi 1, 2, 3 et 4 ; phényl-(alkyle en $C_1$-$C_4$) dans lequel le groupe phényle est éventuellement substitué à la position ortho, méta et/ou para par 1 à 3 groupes choisis parmi les groupes alkyle en $C_1$-$C_4$, nitro, bromo, chloro, iodo, alcoxy en $C_1$-$C_4$ et phényle ;

$R^6$ représente un atome d'hydrogène ; un groupe alkyle en $C_1$-$C_{12}$ ; cycloalkyle en $C_4$-$C_7$ ; cyano-(alkyle en $C_1$-$C_3$) ; -$(CH_2)_r$-OOC-(alkyle en $C_1$-$C_6$) ; dans lequel r est un entier choisi parmi 1, 2, 3 et 4 ; phényl(alkyle en $C_1$-$C_4$) dans lequel le groupe phényle est éventuellement substitué à la position ortho, méta et/ou para par 1 à 3 groupes choisis parmi les groupes alkyle en $C_1$-$C_4$, nitro, bromo, chloro, iodo, alcoxy en $C_1$-$C_4$ et phényle ; ou bien $R^6$ représente un groupe $[CHR^7(CR^8R^9)_mX]_n$-$R^{10}$

dans lequel :

$R^7$ et $R^8$ représentent indépendamment H ou un groupe alkyle en $C_1$-$C_6$ ;

$R^9$ représente H, un groupe alkyle en $C_1$-$C_6$ ou OH ;

$R^{10}$ représente H, un groupe alkyle en $C_1$-$C_3$, $COOR^{11}$, $OR^{11}$, $SR^{11}$, $NR^{11}R^{12}$ ou un atome d'halogène ;

$R^{11}$ et $R^{12}$ représentent indépendamment H ou un groupe alkyle en $C_1$-$C_3$ ; m est zero ou 1, n est un entier compris entre zéro et 6, y compris les extrêmes.

X est O, NH ou une liaison sous réserve que lorsque X est O ou NH, n est alors différent de zéro, et $R^9$ est différent de OH, sous réserve que lorsqu'un des groupes R et $R^6$ représente un groupe $(CH_2)_n$-OOC-(alkyle en $C_1$-$C_6$), l'autre doit représenter un atome d'hydrogène ;

Y représente un groupe

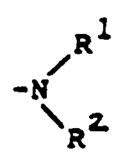

dans lequel

$R^1$ représente un atome d'hydrogène, un groupe alkyle en $C_1$-$C_6$, hydroxy(alkyle en $C_2$-$C_4$),

halogéno(alkyle en $C_2$-$C_4$), alcoxy en $C_1$-$C_4$, alkyle en $C_2$-$C_4$, amino(alkyle en $C_2$-$C_4$), (alkylamino en $C_1$-$C_4$) (alkyle en $C_2$-$C_4$), di(alkylamino en $C_1$-$C_4$)(alkyle en $C_2$-$C_4$) ;

$R^2$ représente un atome d'hydrogène, un groupe alkyle en $C_1$-$C_6$, hydroxy(alkyle en $C_2$-$C_4$), halogéno(alkyle en $C_2$-$C_4$), (alcoxy en $C_1$-$C_4$) (alkyle en $C_2$-$C_4$), un cycle hétérocyclique azoté à 5-6 chaînons qui peut être insaturé, partiellement saturé ou complètement saturé et peut contenir 1 à 3 autres hétéroatomes choisis parmi N, S et O, dans lequel 1 à 3 des atomes de carbone du cycle peuvent éventuellement porter des substituants alkyle en $C_1$-$C_4$ et un des cycles azotés peut éventuellement porter un substituant $R^5$ choisi parmi les groupes alkyle en $C_1$-$C_4$, cycloalkyle en $C_4$-$C_7$, phényle éventuellement substitué par un atome d'halogène ou un groupe alkyle en $C_1$-$C_4$, phényl-(alkyle en $C_1$-$C_4$), pyridyle, (alkyle en $C_1$-$C_4$)pyridinio, et lorsque le cycle est totalement saturé, deux des éléments du cycle peuvent éventuellement être pontés par une chaîne alkylène en $C_1$-$C_3$ dans laquelle un des groupes méthylène peut éventuellement être remplacé par -NH- ou -N(alkyle en $C_1$-$C_4$) ; un groupe -alk-W dans lequel "alk" représente une chaîne alkylène linéaire en $C_1$-$C_8$ qui est éventuellement substituée par un substituant choisi parmi un groupe alkyle en $C_1$-$C_4$, hydroxy(alkyle en $C_1$-$C_4$), hydroxy, carboxyaminocarbonyle, ($C_1$-$C_4$)alkylaminocarbonyle, di($C_1$-$C_4$)alkylaminocarbonyle, ($C_1$-$C_4$)alkylcarbonyle, phényl($C_1$-$C_4$)alcoxycarbonyle et W représente un groupe carboxy, ($C_1$-$C_4$)-alcoxycarbonyle, phényl($C_1$-$C_4$)alcoxycarbonyle, aminocarbonyle, ($C_1$-$C_4$)alkylaminocarbonyle, di($C_1$-$C_4$)alkylaminocarbonyle, pentosamino-carbonyle, hexosaminocarbonyle, uréido, guanidino, un cycle hétérocyclique azoté à 5-6 chaînons tel que défini ci-dessus, un groupe répondant à la formule

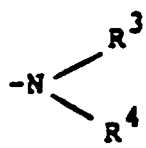

dans laquelle $R^3$ et $R^4$ représentent chacun indépendamment un atome d'hydrogène, un groupe alkyle en $C_1$-$C_6$, hydroxy(alkyle en $C_2$-$C_4$) et halogéno(alkyle en $C_2$-$C_4$), ou bien $R^4$ représente un groupe phénylméthyloxycarbonyle et $R^3$ représente un atome d'hydrogène ; ou bien $R^1$ et $R^2$ représentent conjointement avec l'atome d'azote adjacent un cycle hétérocyclique saturé à 5-7 chaînons qui peut éventuellement porter un à deux substituants alkyle en $C_1$-$C_4$ sur les atomes de carbone du cycle et qui peut contenir un autre hétérogroupe choisi parmi -O-, -S-, et -$NR^5$- dans lequel $R^5$ est tel que défini ci-dessus ;

A représente un atome d'hydrogène ou un groupe -N(acyle aliphatique en $C_9$-$C_{12}$)-bêta-D-2-désoxy-2-aminoglucopyranosyle ;

B représente un atome d'hydrogène ou un groupe N-acétyl-bêta-D-2-désoxy-2-aminoglucopyranosyle ;

M représente un atome d'hydrogène ou un groupe alpha-D-mannopyranosyle ;

sous réserve que B ne représente un atome d'hydrogène que lorsque A et M sont simultanément des atomes d'hydrogène, et sous réserve supplémentaire que lorsque W représente un groupe

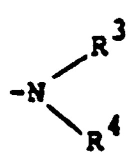 ,

uréido, guanidino ou un cycle hétérocyclique azoté à 5-6 chaînons tel que défini ci-dessus, relié directement à la partie "alk" par une liaison avec un atome d'azote du cycle, la partie alkylène linéaire "alk" doit être d'au moins deux atomes de carbone ; et ses sels d'addition pharmaceutiquement acceptables, qui comprend le fait de faire réagir un composé répondant à la formule II

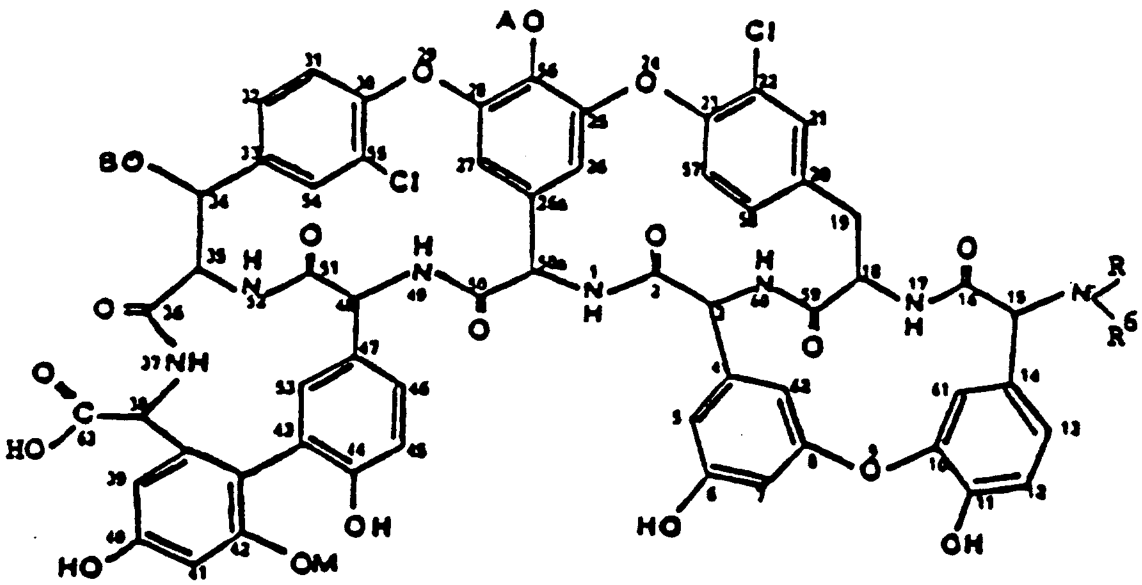

II

dans laquelle :

A, B, M, R et $R^6$ sont tels que définis, protégé sur la fonction 15-amino par un groupe $N^{15}$-protecteur lorsqu'au moins un des groupes R et $R^6$ est H, avec un réactif formant un ester activé pour donner une fonction ester activé qui est substituée par une amine répondant à la formule $NHR^1R^2$ dans laquelle $R^1$ et $R^2$ sont tels que définis, par contact avec un excès de cette amine dans un solvant organique polaire à une température comprise entre 5 ° C et 60 ° C.

**2.** Procédé selon la revendication 1, dans lequel le groupe $N^{15}$-protecteur est choisi parmi des réactifs formant des carbamates caractérisés par les groupes oxycarbonyle suivants : 1,1-diméthylpropynyloxycarbonyle, t-butyloxycarbonyle, vinyloxycarbonyle, aryloxycarbonyle, cinnamyloxycarbonyle, benzyloxycarbonyle, p-nitrobenzyloxycarbonyle, 3,4-diméthoxy-6-nitrobenzyloxycarbonyle, 2,4-dichlorobenzyloxycarbonyle, 5-benzisoxazolylméthyloxycarbonyle, 9-anthranylméthyloxycarbonyle, diphénylméthyloxycarbonyle, isonicotinyloxycarbonyle, S-benzyloxycarbonyle ou des aldéhydes et cétones qui sont capables de former des bases de Schiff avec le groupe $N^{15}$-amino de la partie téicoplanine.

**3.** Procédé selon la revendication 2, dans lequel les groupes $N^{15}$-protecteurs sont choisis parmi les groupes benzyloxycarbonyle et t-butyloxycarbonyle.

**4.** Procédé selon les revendications 1, 2 ou 3, dans lequel les réactifs formant un ester activé sont choisis parmi $ClCH_2CN$, $BrCH_2COOC_2H_5$, $BrCH(COOC_2H_5)_2$, $ClCH_2COCH_3$,

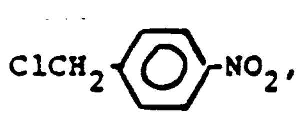, $ClCH_2CH_2N(C_2H_5)_2$.

**5.** Procédé selon l'une quelconque des revendications 1 à 4, dans lequel le réactif formant un ester activé est $ClCH_2CN$.

**6.** Procédé selon l'une quelconque des revendications 1 à 5, dans lequel le solvant organique polaire est choisi parmi des alcanols en $C_1$-$C_4$, le N,N-diméthylformamide,l'hexaméthylphosphoramide, la 1,3-diméthyl-3,4,5,6-tétrahydro-2(1H)-pyrimidinone (DMPU), le diméthylsulfoxyde, le diméthoxyéthane ou des mélanges de ceux-ci.

**7.** Procédé selon l'une quelconque des revendications 1 à 6, caractérisé en outre en ce que le groupe $N^{15}$-protecteur est éliminé à la fin de la réaction de condensation par hydrogénation catalytique ou par un déplacement sélectif dans des conditions acides qui n'affectent pas les autres fonctions présentes dans le dérivé amide désiré.

**8.** Procédé selon la revendication 7, dans lequel l'hydrogénation catalytique est effectuée en présence de palladium sur carbone.

**9.** Procédé selon la revendication 7, dans lequel le déplacement sélectif est effectué en présence d'acide trifluoracétique anhydre à la température ambiante.

**10.** Procédé selon l'une quelconque des revendications 1 à 9, dans lequel l'excès du réactif aminé est compris entre 1:5 et 1:40 par rapport au dérivé ester activé.

**11.** Procédé selon la revendication 10, dans lequel l'excès du réactif aminé est de 1 à 10 par rapport au dérivé ester activé.

**12.** Procédé selon l'une quelconque des revendications 1 à 11 pour préparer un composé répondant à la formule I dans laquelle :

a) A est un groupe N-(acyle aliphatique en $C_9$-$C_{11}$)-bêta-D-2-désoxy-2-aminoglucopyranosyle ;
B est un groupe N-acétyl-bêta-D-2-désoxy-2-aminoglucopyranosyle ;
M est un groupe alpha-D-mannopyranosyle ;
R et $R^6$ sont des atomes d'hydrogène et
Y est $NH_2$, $NHCH_3$, $NH(CH_2)_2NH_2$, $NH(CH_2)_3NH_2$, $NH(CH_2)_2$-$NHCH_3$, $NH(CH_2)_3NHCH_3$, $NH(CH_2)_2NH(C_2H_5)$, $NH(CH_2)_3$-$NH(C_2H_5)$, $N(CH_3)(CH_2)_2NHCH_3$, $N(CH_3)(CH_2)_3NHCH_3$, $NHCH(COOCH_3)$-$(CH_2)_4NH_2$, $NH(CH_2)_3NH(CH_2)_2OH$ ou $NH(CH_2)_3N(CH_3)_2$ ;

b) A est un groupe N-(acyle aliphatique en $C_9$-$C_{11}$)-bêta-D-2-désoxy-2-aminoglucopyranosyle ;
B est un groupe N-acétyl-bêta-D-2-désoxy-2-aminoglucopyranosyle ;
M est un groupe alpha-D-mannopyranosyle
R est un atome d'hydrogène, $R^6$ est $CH_2CH_2$-$N(CH_3)_2$ et
Y est $NH(CH_2)_3N(CH_3)_2$ ;

c) A est un groupe N-(8-méthylnonanoyl)-bêta-D-2-désoxy-2-aminoglucopyranosyle ;
B est un groupe N-acétyl-bêta-D-2-désoxy-2-aminoglucopyranosyle ;
M est un groupe alpha D-mannopyranosyle ;
R et $R^6$ sont des atomes d'hydrogène et
Y est $NH(CH_2)_3$-$N(CH_3)_2$ ;

d) A, B, M, R et $R^6$ sont des atomes d'hydrogène et
Y est $NH_2$, $NHCH_3$, $NH(CH_2)_2NH_2$, $NH(CH_2)_3NH_2$, $NH(CH_2)_4$-$NH_2$, $NH(CH_2)_6NH_2$, $NH(CH_2)_2NHCH_3$, $NH(CH_2)_3NHCH_3$, $NH(CH_2)_2NHC_2H_5$, $NH(CH_2)_5N(CH_3)_2$, $NH(CH_2)_7N(CH_3)_2$, $N(CH_3)(CH_2)_2NHCH_3$, $N(CH_3)(CH_2)_3NHCH_3$ et $NH(CH_2)_3$-$NH(CH_2)_2OH$ ;

e) A, B, M et R sont des atomes d'hydrogène, $R^6$ est $CH(CH_3)CH(CH_3)OH$ et Y est $NH(CH_2)_3$-$N(CH_3)_2$ ;

et ses sels pharmaceutiquement acceptables.

**13.** Procédé selon l'une quelconque des revendications 1 à 11 pour préparer le composé répondant à la formule I qui est la $C^{63}$ 3-diméthylamino-1-propylamidetéicoplanine $A_2$ constituant 2, et ses sels pharmaceutiquement acceptables.